# EUROPEAN PATENT APPLICATION

(11) **EP 2 601 981 A1**
(43) Date of publication of application: **12.06.2013**
(21) Application number: 12194076.1
(22) Date of filing: 29.08.2008
(51) Int. Cl.: A61L 15/60, C08F 8/00, C08F 20/00, A61K 9/16, A61K 31/78, C08J 3/12, C08F 220/06, A61K 9/48, C08F 222/10

(54) **Absorbent polymeric compositions with varying counterion content and their methods of preparation and use**

(30) Priority: 29.08.2007 US 968818 P; 29.08.2007 US 968820 P; 29.08.2007 US 968821 P
(62) Divisional of application: 08798991.9
(71) Applicant: Sorbent Therapeutics, Inc., Vernon Hills, IL 60061 (US)
(72) Inventor: Strickland, Alan, Lake Jackson, TX Texas 77566 (US); Grass, George, Tahoe City, CA California 96145 (US)
(74) Representative: Huenges, Martin

(57) **Abstract**

Cross-linked polyelectrolyte polymers with bound counterions that absorb about 20-fold or more of their mass in saline such as physiological saline a with the proviso that sodium does not exceed 60% of total bound counterions when hydrogen is the sole other counterion, are disclosed. Methods of preparing the disclosed polymers and for treating subjects such as patients in need of fluid removal and/or modulation of ions (e.g., sodium and/or potassium) are provided.

## Description

### FIELD OF THE DISCLOSURE

The present disclosure relates generally to compositions comprising cross-linked polyelectrolyte polymers with bound counterions, including those that absorb about 20-fold, 40-fold or more of their mass in saline. The present disclosure also relates generally to compositions comprising cross-linked polyelectrolyte polymeric beads with bond counterions with the proviso that sodium does not exceed 60% of total bound counterions when hydrogen is the sole other counterion. The present disclosure also relates generally to methods of preparing cross-linked polyelectrolyte polymers with varying counterion content, including those that absorb about 20-fold, 40-fold or more of their mass in saline. The present disclosure also relates generally to methods for treating subjects, including, for example, (i) modulating the levels of one or more ions in a subject, (ii) removing fluid from a subject, and/or (iii) removing waste products from a subject. The present disclosure also relates to methods for treating subjects with diseases or disorders associated with ion imbalances and/or an increased retention of fluid, including methods for treating end stage renal disease (ESRD), chronic kidney disease (CKD), congestive heart failure (CHF) or hypertension.

### BACKGROUND

Numerous diseases and disorders are associated with ion imbalances (e.g., hyperkalemia, hypercalcemia, hyperphosphatemia and hyperoxalemia) and/or increased retention of fluid (e.g., congestive heart failure and end stage renal disease (ESRD)). For example, patients afflicted with an increased level of potassium may exhibit a variety of symptoms ranging from malaise, palpitations, muscle weakness and in severe cases, cardiac arrhythmias. Also, for example, patients afflicted with increased levels of sodium (e.g., hypematremia) may exhibit a variety of symptoms including, lethargy, weakness, irritability, edema and in severe cases, seizures and coma. Additionally, patients affflicted with retention of fluid often suffer from edema (e.g. pulmonary edema and/or edema of the legs) and the buildup of waste products in the blood (e.g., urea, creatinine, other nitrogenous waste products, and electrolytes or minerals, such as sodium, phosphate and potassium).

Treatments for diseases or disorders associated with ion imbalances and/or an increased retention of fluid attempt to restore the ion balance and decrease the retention of fluid. For example, treatment of diseases or disorders associated with ion imbalances may employ the use of ion exchange resins to restore ion balance. Treatment of diseases or disorders associated with an increased retention of fluid may involve the use of diuretics (*e.g*., administration of diuretic agents and/or dialysis, such as hemodialysis or peritoneal dialysis and remediation of waste products that accumulate in the body. Additionally or alternatively, treatment for ion imbalances and/or increased retention of fluid may include restrictions on dietary consumption of electrolytes and water. However, the effectiveness and/or patient compliance with present treatments is less than desired.

### SUMMARY

Compositions of cross-linked polyelectrolyte polymers with bound counterions, including cross-linked polyelectrolyte polymeric beads with bound counterions, along with methods of making and using such polymers are provided.

The present disclosure provides compositions comprising cross-linked polyelectrolyte polymeric beads with bound counterions, including those that absorb about 20-fold, 40-fold or more of their mass in a saline solution, with the proviso that when the bound counterions consist of sodium and hydrogen, the bound sodium counterions are less than 60% while the bound hydrogen ions are greater than 40% of the total bound counterions.

Counterions may include inorganic and/or organic counterions. For example, inorganic counterions include hydrogen, sodium, potassium, calcium, magnesium and/or ammonium while organic counterions may include choline, arginine and/or lysine.

The present disclosure also provides methods of modulating levels of more than one ion in a subject by obtaining one or more compositions of the present disclosure and administering the composition to the subject.

The composition may bind to one or more ions in the subject thereby decreasing the levels of one or more ions in the subject. Alternatively, the composition may release one or more ions in the subject thereby increasing the levels (e.g., total body, serum, fecal and/or urinary levels) of one or more ions in the subject. In some embodiments, the composition may bind to one or more ions in the subject thereby decreasing the levels of one or more ions in the subject and the composition may release one or more ions in the subject thereby increasing the levels of one or more ions in the subject.

The present disclosure also provides methods of removing fluid from a subject by obtaining one or more compositions of the present disclosure and administering the composition to the subject.

In some embodiments, the fluid may be removed from the gastrointestinal tract (e.g., small intestine). Optionally, one or more agents may be administered (together or separately from the polymer) to increase the amount of fluid in the intestine.

The present disclosure also provides methods of removing one or more waste products from a subject by obtaining one or more compositions of the present disclosure, administering the composition to the subject.

The present disclosure also provides methods of preparing cross-linked polyelectrolyte polymeric beads that absorb about 20-fold, 40-fold or more of their mass in a saline solution by obtaining polyelectrolyte polymeric beads, washing the particles with an acid until 100% of bound counterions on the beads are hydrogen and drying the washed beads.

In an embodiment, the polymers, including polymeric beads, may be coated with various coatings. In an embodiment the polymers may be substantially coated, such as with enteric coatings or delayed release coatings. In an embodiment, the polymers may be in dosage forms that are coated, including where the dosage forms are substantially coated, such as with enteric coatings or delayed release coatings. Methods for treating subjects such as patients are also disclosed. With certain disclosed embodiments, subjects having fluid overload conditions are treated. With certain disclosed embodiments, subjects that are in need of sodium removal (e.g., reduced sodium) are also treated. The methods include administering the disclosed cross-linked polyelectrolyte polymers to the intestine of subjects selected for such administration. In an embodiment, the polymer may be administered to specific areas of the GI tract. For example, the polymer may be directed to the intestinal tract (e.g., jejunum). Polymers may be administered orally.

Methods for preparing cross-linked polyelectrolyte polymers, including cross-linked polyelectrolyte polymeric beads, are also disclosed. The methods may include obtaining a cross-linked polyelectrolyte in a particle form from a suspension polymerization reaction, including from an inverse suspension, with a cross-linker, collecting the particles, washing the particles with an acid to remove counterions other than hydrogen, including until substantially free of such counterions, and then drying the washed particles until they may absorb at least 20 times, 40 times or more their mass in a saline solution (e.g., physiological saline), wherein 100% of the total bound counterions are hydrogen. The dried, washed particles may be neutralized by mixing a solution comprising counterions other than hydrogen (e.g., sodium, potassium, magnesium, calcium, ammonium, choline and/or lysine) with the particles to produce particles with desired counterion content.

Additional features and advantages are described herein, and will be apparent from, the following Detailed Description and the Figures.

### BRIEF DESCRIPTION OF THE FIGURES

Figure 1 shows sodium output in rat urine as a function of sodium loading in administered CLP beads, as described in Example 3.

Figure 2 shows sodium output in rat feces as a function of sodium loading in administered CLP beads, as described in Example 3.

Figure 3 shows the swelling amounts as a function of time for various sodium loaded CLP compositions.

### DETAILED DESCRIPTION

Cross-linked polyelectrolyte polymers including, cross-linked polyelectrolyte polymeric beads such as cross-linked polyacrylate polymeric beads, that absorb about 20-fold, 40-fold or more of their mass in a saline solution and with varying counterion content are disclosed. These polymers may contain varying amounts of bound counterions, including, for example, cations such as sodium and/or potassium). Polyelectrolytes have multiple ion binding sites that are occupied (or calculated to be occupied) by counterions. For example, polyacrylates have multiple ion binding sites that are carboxylic acid groups to which counterions are bound. As described herein, cross-linked polyelectrolyte polymers including, cross-linked polyelectrolyte polymeric beads such as cross-linked polyacrylate polymeric beads, have been prepared with varying counterion content, for example, 95% hydrogen bound counterions and 5% sodium bound counterions. Such a polymer with bound hydrogen counterions that are 95% of the total bound counterions indicates that 95% of the ion binding sites of the electrolyte are bound (or calculated to be bound) to hydrogen. Surprisingly, it has been discovered that the polyelectrolyte polymers of the present disclosure may bind and/or release one or more ions (e.g., hydrogen, sodium, potassium, magnesium, calcium, ammonium, choline, arginine and/or lysine) when administered to subjects, including human subjects. Accordingly, these polyelectrolyte polymers may be used to bind, including remove ions, such as sodium, potassium, and/or calcium from a subject. Additionally or alternatively, the polyelectrolyte polymer may release one or more bound ions into the gastrointestinal tract (e.g., small intestine) to increase levels of one or more ions (e.g., where there is a deficiency in one or more ions).

Compositions are provided that comprise cross-linked polyelectrolyte polymeric beads with bound counterions that absorb about 20-fold, 40-fold or more of their mass in a saline solution, with the proviso that when the bound counterions consist of sodium and hydrogen, the bound sodium counterions are less than 60% while the bound hydrogen ions are greater than 40% of the total bound counterions. Optionally, the cross-linked polyelectrolyte polymer, including cross-linked polyelectrolyte polymeric beads, may be disrupted into smaller particles (e.g., by grinding or milling).

The saline holding capacity of the polyelectrolyte polymers may be determined *in vitro.* It is preferred that the *in vitro* saline binding capacity is determined in conditions that mimic the physiological conditions of the gastro-intestinal tract. The disclosed polyelectrolyte polymers preferably absorb 20-fold, 40-fold or more of their mass in a saline solution. The saline solutions for such determinations preferably have a sodium concentration of 0.15M as determined *in vitro.*

In some embodiments of cross-linked polyelectrolyte polymers, including polymeric beads, with varying counterion content, the counterion is an inorganic counterion. In further embodiments, the inorganic counterion is selected from the group consisting of: hydrogen, sodium, potassium, calcium, magnesium and ammonium.

In some embodiments, the counterion is an organic counterion. In further embodiments, the organic counterion is selected from the group consisting of: choline and lysine.

In some embodiments, at least one counterion is an inorganic counterion and at least one counterion is an organic counterion.

In some embodiments, the inorganic counterion is hydrogen. In some embodiments, hydrogen comprises from greater than 40% to 100% of the total bound counterions. In some embodiments, hydrogen comprises 100% of the total bound counterions. In some embodiments, hydrogen comprises 75% of the total bound counterions. In some embodiments, hydrogen comprises 50% of the total bound counterions. In some embodiments, hydrogen comprises 25% of the total bound counterions.

In some embodiments, the inorganic counterion is sodium. In some embodiments, sodium comprises 50% of the total bound counterions. In some embodiments, sodium comprises 25% of the total bound counterions.

In some embodiments, the inorganic counterion is potassium. In some embodiments, potassium comprises 100% of the total bound counterions. In some embodiments, potassium comprises 75% of the total bound counterions. In some embodiments, potassium comprises 50% of the total bound counterions. In some embodiments, potassium comprises 25% of the total bound counterions.

In some embodiments, the inorganic counterion is ammonium. In some embodiments, ammonium comprises 100% of the total bound counterions. In some embodiments, ammonium comprises 75% of the total bound counterions. In some embodiments, ammonium comprises 50% of the total bound counterions. In some embodiments, ammonium comprises 25% of the total bound counterions.

In some embodiments, the organic counterion is choline. In some embodiments, choline comprises 100% of the total bound counterions. In some embodiments, choline comprises 75% of the total bound counterions. In some embodiments, choline comprises 50% of the total bound counterions. In some embodiments, choline comprises 25% of the total bound counterions.

In some embodiments, the organic counterion is lysine. In some embodiments, lysine comprises 100% of the total bound counterions. In some embodiments, lysine comprises 75% of the total bound counterions. In some embodiments, lysine comprises 50% of the total bound counterions. In some embodiments, lysine comprises 25% of the total bound counterions.

In some embodiments, the counterions are hydrogen and sodium. In some embodiments, hydrogen comprises from greater than 40% to 100% of total bound counterion and sodium comprises from greater than 0 to less than 60% of total bound counterion.

In some embodiments, the counterions are hydrogen and potassium. In some embodiments, the counterions are sodium and potassium. In some embodiments, the counterions are sodium, potassium and hydrogen. In some embodiments, the counterions are hydrogen and ammonium. In some embodiments, the counterions are hydrogen and choline. In some embodiments, the counterions are hydrogen and lysine.

In some embodiments, the polymer, including polymeric beads are substantially coated with a coating. In some embodiments, the beads whether coated or uncoated are surrounded by a capsule. In some embodiments, the capsule is coated with a coating. In some embodiments, the coating is an enteric or delayed release coating.

In some embodiments, the polyelectrolyte is polyacrylate.

Methods are provided for modulating levels of one or more, including more than one ion, in a subject by obtaining a composition of the present disclosure, administering the composition to the subject; and modulating the levels of one or more, including more than one, ion in the subj ect.

In some embodiments, the methods may further comprise identifying a subject in need of modulation of more than one ion.

In some embodiments, the modulation is an increase or decrease in the level of one or more ions.

In some embodiments, the composition binds to and removes one or more ions in the subject thereby decreasing the levels of one or more ions in the subject. In some embodiments, the composition releases one or more ions in the subject thereby increasing the levels of one or more ions in the subject. In some embodiments, the composition binds to one or more first ions in the subject thereby decreasing the levels of one or more first ions in the subject and the composition releases one or more second ions in the subject thereby increasing the levels of one or more second ions in the subject.

In some embodiments, the composition removes sodium. In some embodiments, the composition removes sodium and releases potassium. In some embodiments, the composition removes sodium and removes potassium. In some embodiments, the composition removes sodium and removes potassium without releasing hydrogen.

In some embodiments, the cross-linked polyelectrolyte polymer is directly administered to the colon. In some embodiments, the cross-linked polyelectrolyte polymer is directly administered to the small intestine. In some embodiments, the polymer is directly administered to the jejunum.

Methods are also provided for removing fluid from a subject by obtaining a composition of the present disclosure, administering the composition to the subject and removing fluid from the subject.

In some embodiments, the methods may further comprise identifying a subject in need of removal of the fluid.

In some embodiments, the methods may further comprise administering to the subject one or more agents that increase the amount of fluid in the intestine.

In some embodiments, the agent is selected from the group consisting of: mannitol, polyethylene glycol and lubiprostone. In some embodiments, the polyethylene glycol has a molecular weight between 400 and 10,000 Daltons. In some embodiments, the polyethylene glycol has a molecular weight between 400 and 4000 Daltons.

In some embodiments, the composition is directly administered to the colon. In some embodiments, the composition is directly administered to the small intestine. In some embodiments, the composition is directly administered to the jejunum.

In some embodiments, the composition is administered orally.

In some embodiments, the subject has cardiac disease. In some embodiments, the cardiac disease is congestive heart failure and/or hypertension. In some embodiments, the subject has kidney disease. In some embodiments, the kidney disease is nephrosis, nephritis, chronic kidney disease (CKD), or end stage renal disease (ESRD). In some embodiments, the subject has an intestinal or nutritional disorder. In some embodiments, the nutritional disorder is kwashiorkor or gluten-sensitive enteropathy. In some embodiments, the subject has hepatic disease. In some embodiments, the hepatic disease is cirrhosis of the liver. In some embodiments, the subject has an endocrine, neurological or immune system disorder. In some embodiments, the endocrine disorder is preclampsia or eclampsia. In some embodiments, the neurological disorder is angioneurotic edema.

Methods are provided for removing one or more waste products from a subject by obtaining a composition of the present disclosure, administering the composition to the subject and removing an amount of one or more waste products from the subject.

In some embodiments, the methods may further comprise identifying a subject in need of removal of one or more waste products;

In some embodiments, the waste product is a metabolic waste. In some embodiments, the metabolic waste is urea, uric acid, creatinine, sodium or potassium.

In some embodiments, the methods may further comprising administering to the subject one or more agents that increase the amount of fluid in the intestine.

In some embodiments, the agent is selected from the group consisting of: mannitol, polyethylene glycol and lubiprostone.

In some embodiments, the composition is directly administered to the colon. In some embodiments, the composition is directly administered to the small intestine. In some embodiments, the composition is directly administered to the jejunum.

In some embodiments, the composition is administered orally.

Methods are also provided for preparing cross-linked polyelectrolyte polymeric beads with 100% bound hydrogen counterion that absorb about 20-fold, 40-fold or more of their mass in a saline solution by obtaining a cross-linked polyelectrolyte polymeric beads, washing the particles with an acid until 100% of bound counterions on the beads are hydrogen and drying the washed beads.

In some embodiments, the polyelectrolyte is polyacrylate.

In some embodiments, the methods may further comprise the step of disrupting the dried beads by milling.

In some embodiments, the methods may further comprise the step of mixing the intact or disrupted beads with a solution comprising counterions other than hydrogen. In some embodiments, the counterions are selected from hydrogen, sodium, potassium, magnesium, calcium, ammonium, choline and lysine.

In some embodiments, the methods may further comprise the step of washing the intact or disrupted beads.

In some embodiments, the methods may further comprise the step of drying the washed intact or disrupted beads

In some embodiments, the beads are substantially coated. In some embodiments, the beads are surrounded by a capsule. In some embodiments, the capsule is coated with a coating. In some embodiments, the coating is an enteric or delayed release coating.

In some embodiments, the cross-linked polyelectrolyte polymers will resist acidic conditions, including, for example, the acidic conditions present in a gastrointestinal tract.

Methods are provided for preparing cross-linked polyelectrolyte beads by polymerizing cross-linked electrolyte monomers neutralized with sodium; obtaining cross-linked polyelectrolyte polymeric beads with bound sodium counterions from the polymerization and reducing the percentage of bound sodium counterions on the beads.

Methods are also provided for preparing cross-linked polyelectrolyte polymeric beads with less than 60% bound sodium counterion by preparing cross-linked polyelectrolyte polymeric beads with greater than 60% bound sodium counterions and reducing the bound sodium counterions to less than 60%.

In some embodiments, the methods further comprise disrupting the beads. In some embodiments, the disrupted beads have a particle size of 212-500 microns.

In some embodiments, the method further comprise encapsulating the disrupted beads in a capsule.

In some embodiments, the monomers are acrylic acid. In some embodiments, the polyelectrolyte is polyacrylate.

In some embodiments, the monomers are neutralized from about 60% to about 100% with sodium. In some embodiments, the monomers are about 80% neutralized with sodium.

In some embodiments, the polymerization is by a method of inverse suspension polymerization.

In some embodiments, the bound sodium counterions are reduced to less than 60%. In some embodiments, the bound sodium counterions are reduced to less than 50%. In some embodiments, the bound sodium counterions are reduced to less than 25%. In some embodiments, the bound sodium counterions are reduced to less than 10%. In some embodiments, the bound sodium counterions are reduced to less than 5%. In some embodiments, the bound sodium counterions are reduced to less than 1%.

In some embodiments, the bound sodium counterions are reduced by acid treatment of the beads. In some embodiments, the acid treated beads are washed to remove excess acid. In some embodiments, the washed beads are dried.

In some embodiments, the beads are encapsulated in a capsule. In some embodiments, the capsule is substantially coated with a coating. In some embodiments, the beads are substantially coated with a coating.

In some embodiments, the beads comprise disrupted beads.

Cross-linked polyelectrolyte polymeric beads are provided with bound counterions comprising 5% or less sodium bound counterions.

Cross-linked polyelectrolyte polymeric beads are also provided with bound counterions comprising 1% or less sodium bound counterions.

Methods of treating a patient with end stage renal disease (ESRD) are provided that comprise administering to a patient with ESRD cross-linked polyelectrolyte polymeric beads wherein the beads comprise less than 60%, less than 50%, less than 25%, less than 10%, less than 5% or less than 1% bound sodium.

Methods of treating a patient with congestive heart failure (CHF) are provided that comprise administering to a patient with CHF cross-linked polyelectrolyte polymeric beads wherein the beads comprise less than 60%, less than 50%, less than 25%, less than 10%, less than 5% or less than 1% bound sodium.

Methods of treating a patient with chronic kidney disease (CKD) are provided that comprise administering to a patient with CKD cross-linked polyelectrolyte polymeric beads wherein the beads comprise less than 60%, less than 50%, less than 25%, less than 10%, less than 5% or less than 1% bound sodium.

Methods of treating a patient with hypertension are provided that comprise administering to the patient with hypertension cross-linked polyelectrolyte polymeric beads wherein the beads comprise less than 60%, less than 50%, less than 25%, less than 10%, less than 5% or less than 1% bound sodium.

Methods of modulating levels of more than one ions in a subject are provided that comprise administering a composition comprising cross-linked polyelectrolyte polymeric beads with bound counterions that absorb at least 20-fold of their mass in a saline solution, with the proviso that when the bound counterions consist of sodium and hydrogen, the bound sodium counterions are less than 60% while the bound hydrogen ions are greater than 40% of the total bound counterions to the subject.

Methods of removing fluid from a subject are provided that comprise administering a composition comprising cross-linked polyelectrolyte polymeric beads with bound counterions that absorb at least 20-fold of their mass in a saline solution, with the proviso that when the bound counterions consist of sodium and hydrogen, the bound sodium counterions are less than 60% while the bound hydrogen ions are greater than 40% of the total bound counterions to the subject.

Method of removing one or more waste products from a subject are provided that comprise administering a composition comprising cross-linked polyelectrolyte polymeric beads with bound counterions that absorb at least 20-fold of their mass in a saline solution, with the proviso that when the bound counterions consist of sodium and hydrogen, the bound sodium counterions are less than 60% while the bound hydrogen ions are greater than 40% of the total bound counterions to the subject.

Compositions are provided that comprise cross-linked polyelectrolyte polymeric beads with bound counterions wherein bound sodium counterions are less than 60% of the total bound counterions.

Compositions are also provided that comprise cross-linked polyelectrolyte polymeric beads with bound counterions wherein bound hydrogen counterions are greater than 40% of the total bound counterions.

Compositions are also provided that comprise cross-linked polyelectrolyte polymeric beads with bound counterions wherein bound sodium counterions are less than 60% of the total bound counterions and bound hydrogen counterions are greater than 40% of the total bound counterions.

### Preparation of Superabsorbent Polyelectrolyte Beads with Varying Counterion Content

Superabsorbent polyelectrolyte beads, including, for example, polyacrylate beads, may be prepared by methods known in the art, including by suspension methods (e.g., Buchholz, F. L. and Graham, A. T., "Modern Superabsorbent Polymer Technology," John Wiley & Sons (1998)). Such methods may include manufacture of polyelectrolyte beads by inverse suspension polymerization. The present disclosure provides novel methods of preparing cross-linked polyelectrolyte polymers, including polymeric beads, with varying counterion content. Such polymers, including polymeric beads have been unexpectedly found to have varying counterion binding and release properties in subjects, including human subjects. Such differential properties make them useful as designer therapeutics for different diseases and conditions involving ion imbalance and/or fluid imbalance. For example, methods are provided for washing the cross-linked polyelectrolyte polymer, including polymeric beads with an acid to replace bound counterions other than hydrogen with hydrogen. Methods are also provided for replacing the bound hydrogen counterions (including 100% bound hydrogen ions) with other counterions. For these methods, beads may be used as disrupted beads by optionally grinding the beads into particles. One form of cross-linked polyelectrolyte consists of polymers containing many carboxylic acid groups which may be reacted with alkali metals to produce polycarboxylates such as polyacrylates. Many of these polycarboxylates act as superabsorbent polymers, absorbing over twenty times their mass in 0.9% saline (0.15 M sodium). Exemplary methods are provided below.

### 1. Manufacture of Superabsorbent Polyelectrolyte

Cross-linked polyelectrolyte polymers, including cross-linked polyelectrolyte polymeric beads, may be prepared by commonly known methods in the art. In an exemplary method, cross-linked polyelectrolyte polymers may be prepared as a suspension of drops of aqueous solution in a hydrocarbon (e.g., by inverse suspension polymerization).

Superabsorbent polyacrylates may be prepared by polymerization of partially neutralized acrylic acid in an aqueous environment where an appropriate cross-linker is present in small quantities. Given that there is an inverse relationship between the amount of fluid the superabsorbent polymer will absorb and the degree of cross-linking of the polymer, it desirable to have the minimum cross-linking possible to still produce a resin. However, there is also an inverse relationship between the degree of cross-linking and the percentage of polymer chains that do not cross-link and are therefore soluble polymer that does not contribute to the absorbency of the resin since it dissolves in the fluid. For example, superabsorbent polyacrylates can be designed to absorb about 35 times their mass in physiological saline as a compromise between maximal absorbency and minimal soluble polymer.

Since the amount of reactants used in an inverse suspension polymerization reaction varies depending upon the size of the reactor, the precise amount of each reactant used in the preparation of cross-linked polyelectrolyte polymer, such as polyacrylate may be determined by one of skill in the art. For example, in a five-hundred gallon reactor, about 190 to 200 pounds (roughly 85 to 90 kg) of acrylic acid may be used while in a three liter reactor 150 to 180 g of acrylic acid may be used. Accordingly, the amount of each reactant used for the preparation of cross-linked polyacrylate are expressed as weight ratios to acrylic acid. Thus, acrylic acid weight is taken as 1.0000 and other compounds are presented in relation to this value. Exemplary amounts of reactants used for the preparation of cross-linked polyacrylate by an inverse suspension polymerization are presented in Table 1.

**Table 1: Exemplary amounts of reactants in an inverse suspension polymerization**

| **Substance** | **Low value** | **High Value** |
|---|---|---|
| Acrylic acid | 1.0000 | 1.0000 |
| Water | 0.5000 | 3.0000 |
| Hydrophobic solvent | 1.2000 | 12.0000 |
| Base (expressed as 50% NaOH) | 0.6600 (60% neutral) | 1.1100 (100% neutralized) |
| Crosslinker | 0.0030 | 0.0080 |
| Initiator | 0.0005 | 0.0200 |
| Chelating agent | 0.0000 | 0.0050 |
| Surfactant | 0.0050 | 0.0400 |

An exemplary inverse suspension reaction to form a superabsorbent polymer may involve preparation of two mixtures *(e.g.,* a hydrophobic and an aqueous mixture) in two different vessels followed by combination of the mixtures to form a reaction mixture. One vessel may be designated as a hydrophobic compound vessel and the other may be designated as an aqueous solution vessel. The hydrophobic compounds may be mixed in a larger vessel that will become a reaction vessel, while an aqueous solution may be prepared in a smaller vessel that may be discharged into the reaction vessel.

A hydrophobic solvent may be introduced into the reaction vessel. As will be appreciated by one of skill in the art, a hydrophobic solvent (also referred to herein as the "oil phase") may be chosen based upon one or more considerations, including, for example, the density and viscosity of the oil phase, the solubility of water in the oil phase, the partitioning of the neutralized and unneutralized ethylenically unsaturated monomers between the oil phase and the aqueous phase, the partitioning of the crosslinker and the initiator between the oil phase and the aqueous phase and/or the boiling point of the oil phase.

Hydrophobic solvents contemplated for use in the present disclosure include, for example, Isopar L, toluene, benzene, dodecane, cyclohexane, n-heptane and/or cumene. Preferably, Isopar L is chosen as a hydrophobic solvent due to its low viscosity, high boiling point and low solubility for neutralized monomers such as sodium acrylate and/or potassium acrylate. One of skill in the art will appreciate that a large enough volume of hydrophobic solvent is used to ensure that the aqueous phase is suspended as droplets in the oil rather than the reverse and that the aqueous phase droplets are sufficiently separated to prevent coalescence into large masses of aqueous phase.

One or more surfactants and one or more crosslinkers may be added to the oil phase. The oil phase may then be agitated and sparged with an inert gas, such as nitrogen or argon to remove oxygen from the oil phase. It will be appreciated that the amount of surfactant used in the reaction depends on the size of the desired beads and the agitator stir rate. This addition of surfactant is designed to coat the water droplets formed in the initial reaction mixture before the reaction starts. Higher amounts of surfactant and higher agitation rates produce smaller droplets with more total surface area. It will be understood by those of skill in the art that an appropriate choice of cross-linker and initiator may be used to prepare spherical to ellipsoid shaped beads. One of skill in the art will be capable of determining an appropriate cross-linker for the preparation of a specified cross-linked polyelectrolyte. For example, cross-linker choice depends on whether it needs to be hydrophobic or hydrophilic or whether it needs to resist acidic or basic external conditions. An amount of cross-linker depends on how much soluble polymer is permissible and how much saline holding capacity is needed.

Exemplary surfactants include hydrophobic agents that are solids at room temperature, including, for example, hydrophobic silicas (such as Aerosil or Perform-O-Sil) and glycolipids (such as polyethylene glycol distearate, polyethylene glycol dioleate, sorbitan monostearate, sorbitan monooleate or ocytl glucoside).

Crosslinking agents with two or more vinyl groups that are not in resonance with each other may be used, allowing for a wide variety in molecular weight, aqueous solubility and/or lipid solubility. Crosslinking agents contemplated for use in the present disclosure, include, for example, diethelyeneglycol diacrylate (diacryl glycerol), triallylamine, tetraallyloxyethane, allylmethacrylate, 1,1,1-trimethylolpropane triacrylate (TMPTA), and divinylbenzene.

An aqueous phase mixture may be prepared in another vessel *(e.g.,* a vessel that is separate from that used to prepare the hydrophobic phase) by placing water into the vessel and adding a base to the water. It will be appreciated by one of skill in the art that the amount of base used in the vessel is determined by the degree of neutralization of the monomer desired. A degree of neutralization between 60% and 100% is preferred. Without wishing to be bound by a theory of the disclosure, it is believed that one-hundred percent neutralization minimizes the chance of suspension failure, but the highly charged monomer may not react as rapidly and may not pull hydrophobic crosslinkers into the beads. Considerations in choosing the degree of neutralization may be determined by one of skill in the art and include, for example, the effect of monomer charge *(e.g.,* as determined by ionization of the cation from the neutralized molecules) on reaction rate, partitioning of the monomer and neutralized monomer between oil phase and aqueous phase and/or tendency to coalescence of the polymer chains during the reaction. The solubilities of sodium acrylate and sodium methacrylate in water are limited and are lower at lower temperatures *(e.g.,* sodium acrylate is soluble at about 45% at 70°C but less than 40% at 20 °C). This solubility may establish the lower limit of the amount of water needed in the neutralization step. The upper limit of the amount of water may be based on reactor size, amount of oil phase needed to reliably suspend the aqueous phase as droplets and/or the desired amount of polymer produced per batch.

Bases contemplated for use in the present disclosure include, for example, hydroxides, bicarbonates, or carbonates. Use of these bases allows neutralization of the acid monomer without residual anions left in the reaction mixture. It will be apparent to one of skill in the art that the cation used for the base may be chosen based on the planned use of the superabsorbent polymer. Normally, sodium bases are chosen since the superabsorbent polymers will be used in situations where saline solutions will be encountered. However, potassium bases, ammonium bases, and bases of other cations are contemplated for use in the present disclosure.

The water used in the reaction may be purified water or water from other sources such as city water or well water. If the water used is not purified water, chelating agents may be needed to control metals such as iron, calcium, and magnesium from destroying the initiator. Chelating agents contemplated for use with the present disclosure include, for example, Versenex 80. The amount of chelating agent added to the reaction mixture may be determined by one of skill in the art from a determination of the amount of metal in the water.

Once base is added to the water, the aqueous phase solution may be cooled to remove the heat released from dilution of the base and one or more classes of monomers may be added to react with the base. As will be appreciated by one of skill in the art, the monomers will be neutralized to the degree dictated by the amount of base in the reaction. The aqueous phase solution may be kept cool (e.g., below 35 to 40 °C) and preferably around 20 °C to prevent formation of prepolymer strands, dimers and/or possible premature polymerization.

Monomers are dissolved in water at concentrations of 20-40 wt% and polymerization may subsequently be initiated by free radicals in the aqueous phase. Monomers may be polymerized either in the acid form (pH 2-4) or as a partially neutralized salt (pH 5-7). The amount of water used to dissolve the monomer is minimally set so that all of the monomer (e.g., sodium acrylate) is dissolved in the water rather than crystallizing and maximally set so that there is the smallest volume of reaction mixture possible (to minimize the amount of distillation and allow the maximum yield per batch).

Exemplary monomer units contemplated for use in the present disclosure, include, for example, acrylic acid and its salts, methacrylic acid and its salts, crotonic acid and its salts, tiglinic acid and its salts, 2-methyl-2-butenoic acid (Z) and its salts, 3-butenoic acid (vinylacetic acid) and its salts, 1-cyclopentene carboxylic acid, and 2-cyclopentene carboxylic acid and their salts. Other cross-linked polyelectrolyte superabsorbent polymers may be based on sulfonic acids and their salts, phosphonic acids and their salts, or amines and their salts.

One or more initiators, free radical producers, may be added to the aqueous phase just before the aqueous phase is transferred into the oil phase. As will be appreciated by one of skill in the art, the initiator amounts and type used in the polymerization reaction depend on oil versus water solubility and the need for longer chain lengths. For example, a lower amount of initiator may be used in the polymerization reaction when longer chain lengths are desired.

In some embodiments, the initiator may be a thermally sensitive compound such as persulfates, 2,2'-azobis (2-amidino-propane)-dihydrocholoride, 2,2' -azobis (2-amidino-propane)-dihydrochloride and/or 2,2'-azobis (4-cyanopentanoic acid) persulfate or 2,2'-azobis(4-cyanopentanoic acid). Thermally sensitive initiators have the disadvantage that the polymerization does not begin until an elevated temperature is reached. For persulfates, this temperature is approximately 50 to 55 °C. Since the reaction is highly exothermic, vigorous removal of the heat of reaction is required to prevent boiling of the aqueous phase. It is preferred that the reaction mixture be maintained at approximately 65 °C. As will be appreciated by one of skill in the art, thermal initiators have the advantage of allowing control of the start of the reaction when the reaction mixture is adequately sparged of oxygen.

In some embodiments, the initiator may also be a redox pair such as persulfate/bisulfate, persulfate/thiosulfate, persulfate/ascorbate, hydrogen peroxide/ascorbate, sulfur dioxide/tert-butylhydroperoxide, persulfate/erythorbate, tert-butylhydroperoxide/erythorbate and/or tert-butylperbenzoate/erythorbate. These initiators are able to initiate the reaction at room temperature, thereby minimizing the chance of heating the reaction mixture to the boiling point of the aqueous phase as heat is removed through the jacket around the reactor. However, homogeneous mixing may not accomplished by the time the reaction is initiated and there may be rapid polymerization of the surface of the droplets with much slower polymerization within the bead.

In preferred embodiments, the reaction is not started immediately after the mixing of the aqueous phase into the oil phase in the final reactor because the aqueous phase still has an excessive amount of oxygen dissolved in the water. It will be appreciated by one of skill in the art that an excessive amount of oxygen may cause poor reactivity and inadequate mixing may prevent the establishment of uniform droplet sizes. Instead, the final reaction mixture is first sparged with the inert gas for ten to sixty minutes after all reagents (except the redox pair if that initiator system is used) have been placed in the reactor. The reaction may be initiated when a low oxygen content *(e.g.,* below 15 ppm) is measured in the inert gas exiting the reactor.

It will be appreciated by those of skill in the art that with acrylate and methacrylate monomers polymerization begins in the droplets and progresses to a point where coalescence of the beads becomes more likely (the "sticky phase"). It may be necessary that a second addition of surfactant *(e.g.,* appropriately degassed to remove oxygen) be added during this phase or that the agitation rate be increased. For persulfate thermal initiation, this sticky phase may occur at about 50 to 55 °C. For redox initiation systems, the need for additional surfactant may be lessened by the initial surface polymerization, but if additional surfactant is needed, it should be added as soon as an exotherm is noted.

The reaction may be continued for four to six hours after the peak exotherm is seen to allow for maximal consumption of the monomer into the polymer. Following the reaction, the beads may be isolated by either transferring the entire reaction mixture to a centrifuge or filter to remove the fluids or by initially distilling the water and some of the oil phase *(e.g.,* frequently as an azeotrope) until no further removal of water is possible and the distillation temperature rises significantly above 100 °C followed by isolating the beads by either centrifugation or filtering. The isolated beads are then dried to a desired residual moisture content *(e.g.,* less than 5%).

An exemplary cross-linked polyelectrolyte, polyacrlylate, may be formed by copolymerizing an ethylenically unsaturated carboxylic acid with a multifunctional cross-linking monomer. The acid monomer or polymer may be substantially or partially neutralized with an alkali metal salt such as the hydroxide, the carbonate, or the bicarbonate and polymerized by the addition of an initiator. One such exemplary polymer gel is a copolymer of acrylic acid/sodium acrylate and any of a variety of cross-linkers.

The reactants for the synthesis of exemplary cross-linked polyelectrolyte polymeric beads, such as cross-linked polyacrylate, are provided in Table 2 below. These cross-linked polyelectrolyte polymeric beads may be produced as a one-hundred kilogram batch in a five-hundred gallon vessel.

**Table 2: List of Components Used in the Manufacture of cross-linked Polyacrylate Beads**

| **Component** | **Function** | **Amount/batch (kg)** |
|---|---|---|
| Acrylic Acid | Monomer | 88 |
| Water | Solvent | 90 |
| 50% Sodium Hydroxide | Neutralization of acrylic acid monomer | 79 |
| Naphtha [petroleum], hydrotreated heavy, (Isopar L) | Continuous phase for suspension | As needed |
| Fumed silica (Aerosil R972) | Suspending agent | 0.9 |
| Diethylenetriaminepentaacetic Acid Pentasodium | Control of metal ions in reagents, solvents, or | 0.9 |
| Sodium Persulfate | Polymerization initiator | 0.06 |
| Trimethylolpropane Triacrylate, (TMPTA) | Cross-linking agent | 0.3 |

An exemplary polymerization reaction is shown below.

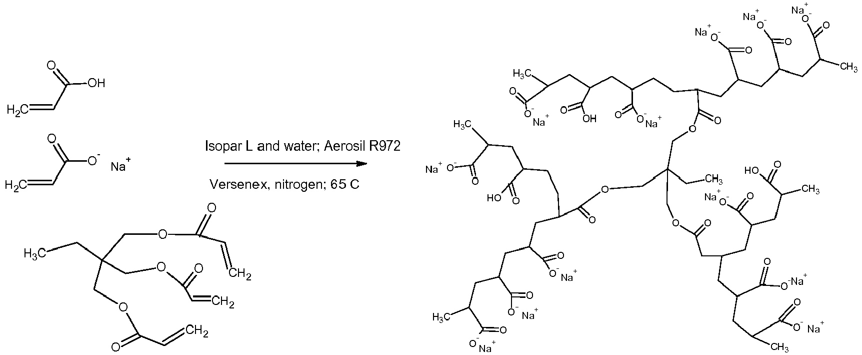

### 2. Preparation of Cross-Linked Polyelectrolyte Polymeric Beads with Hydrogen Counterions

Partially neutralized or non-neutralized polyelectrolyte polymers may be prepared with 100% hydrogen counterion content by washing the polymer with acid. Suitable acids contemplated for use with the present disclosure, include, for example, hydrochloric acid, acetic acid and phosphoric acid.

Those skilled in the art will recognize that the replacement of the counterions, including cations such as sodium atoms, by hydrogen atoms can be performed with many different acids and different concentrations of acid. However, care must be taken in choice of acid and concentration to avoid damage to the polymer or the cross-linkers. For instance, nitric and sulfuric acids would be avoided.

Acid washed polyelectrolyte polymers may then be dried in a vacuum oven or inert atmosphere until less than 5% moisture remains to produce cross-linked polyacrylic acid which is substantially the free acid form of lightly cross-linked polyacrylic acid. Optionally, if the intact bead form of partially-neutralized, lightly cross-linked polyacrylate is used, the cross-linked polyelectrolyte polymer may be left in the bead form recovered from the oven or may be milled to obtain smaller particles of low-sodium cross-linked polyelectrolyte polymer.

### 3. Preparation of Cross-Linked Polyelectrolyte Polymeric Beads with Varying Counterion Content

The free acid form of cross-linked polyelectrolyte polymers of the present disclosure, including, for example, cross-linked polyacrylic acid may be converted into polymer with various levels of one or more counterions (e.g., one or more inorganic counterions, such as sodium, potassium, calcium, magnesium and/or ammonium and/or one or more organic counterions, such as choline and/or lysine). These methods may be carried out with intact beads, with disrupted beads, or with powdered forms of cross-linked polyelectrolyte polymers, including for example, polyacrylate polymers.

Suitable counterions include alkali metals and alkaline earth metals, including, for example, sodium, potassium, calcium or magnesium and exclude hydrogen. Counterions may be selected based on the requirements of an individual patient. For example, by appropriate selection of counterions electrolytic imbalances in patients may be treated. For example, in patients having excess sodium, sodium would be avoided as a counterion.

Counterions may be provided as salts that could be dissolved to a sufficient degree in aqueous solution and mixed with the acid form of the polymer. Particularly advantageous choices of salts would be those that neutralize the acid in such a way as to produce products that are easily removed from the polymer. Such salts include the carbonate salt of the desired counterion (e.g. sodium carbonate, potassium carbonate, calcium carbonate), the bicarbonate salt of the desired counterion (e.g. calcium bicarbonate, magnesium bicarbonate, lithium bicarbonate), or the hydroxide or oxide of the desired counterion (e.g. sodium hydroxide, choline hydroxide, magnesium hydroxide, magnesium oxide).

### 4. Preparation of Cross-Linked Polyelectrolyte Polymeric Beads with Increased Saline Holding Capacity

Partially neutralized or non-neutralized polyelectrolyte polymers of the present disclosure, including cross-linked polyelectrolyte polymeric beads, may be disrupted to increase their saline holding capacity. Saline holding capacity is preferably determined as described in Example 4, wherein the beads or disrupted beads are include with a neutral pH (e.g., pH 7) saline solution having a sodium concentration of 0.15 M. Alternatively, a 0.9% saline solution (0.154 M sodium) may be used.

Cross-linked polyelectrolyte polymeric beads, including cross-linked polyacrylate polymeric beads, may be disrupted into smaller particles, for example, by milling or crushing in a grinder. The disrupted polymeric beads are preferably washed to remove soluble polymer. Suitable washing solutions include purified water such as deionized water or distilled water and various alcohols. Since the polymer is to be dried, it is desirable to use fluids that will evaporate easily without leaving any residue, such as salts, in the dried polymer. Alternatively, cross-linked polyelectrolyte polymeric beads, including cross-linked polyacrylate polymeric beads may be disrupted by placing the beads into purified water and agitating the beads (e.g., stirring with a magnetic stir bar or agitating at 500 rpm overnight), the residual soluble polymer in the polymeric beads may be reduced or eliminated and the saline holding capacity of the polymeric beads increased.

Particles of a certain size, may be obtained by sieving through sieves such as screens. Screens may be stacked to obtain particles with a range of sizes. Screens are shaken to allow particles to sift through and get caught on the screen with an opening just below their diameter. For example, particles that pass through an 18 Mesh screen and are caught on a 20 Mesh screen are between 850 and 1000 microns in diameter. Screen mesh and the corresponding particle size allowed to pass through the mesh include, 18 mesh, 1000 microns; 20 mesh, 850 microns; 25 mesh, 710 microns; 30 mesh, 600 microns; 35 mesh, 500 microns, 40 mesh, 425 microns; 45 mesh, 35 microns; 50 mesh, 300 microns; 60 mesh, 250 microns; 70 mesh, 212 microns; 80 mesh, 180 microns; 100 mesh, 150 microns; 120 mesh, 125 microns; 140 mesh, 106 microns; 170 mesh, 90 microns; 200 mesh, 75 microns; 230 mesh, 63 microns; and 270 mesh, 53 microns. Thus particles of varying sizes may be obtained through the use of one or more screens.

### Therapeutic Uses

The disclosed polymers and compositions comprising the polymers have a variety of uses, including therapeutic uses. Such uses may include methods for the removal of fluid. Such uses may also include methods for treating diseases or disorders associated with increased retention of fluid and/or ion imbalances. The disclosed polymers may be used in methods to treat end stage renal disease (ESRD), chronic kidney disease (CKD), congestive heart failure (CHF) or hypertension. The disclosed polymers may also be used in methods to treat an intestinal disorder, a nutritional disorder (e.g., kwashiorkor or gluten-sensitive enteropathy), a hepatic disease (e.g., cirrhosis of the liver), an endocrine disorder *(e.g.,* preclampsia or eclampsia), a neurological disorder *(e.g.,* angioneurotic edema) or immune system disorder. The discloses polymers may be administered in combination with agents that increase fluid in the intestine *(e.g.,* osmotic agents, irritants, sodium absorption blocking agents and agents that enhance fluid secretion).

The methods may be used to modulate *(e.g.,* increase or decrease) levels of one or more ions, including more than one ion, in a subject by administering a composition of the present disclosure to the subject in an amount effective to modulate the levels of one or more ions, including more than one ion, in the subject.

The composition may bind to one or more ions in the subject thereby decreasing the levels of one or more ions in the subject. Additionally, the composition may release one or more ions in the subject thereby increasing the levels of one or more ions in the subject. Alternatively, the composition may bind to one or more first ions in the subject thereby decreasing the levels of one or more first ions in the subject and the composition release one or more second ions in the subject thereby increasing the levels of one or more second ions in the subject.

The composition may be used to remove one or more ions selected from the group consisting of: hydrogen, sodium, potassium, calcium, magnesium and/or ammonium.

In some embodiments, the polymer may be substantially coated with a coating that allows it to pass through the gut and open in the intestine where the polymer may absorb fluid or specific ions that are concentrated in that particular portion of the intestine. In some embodiments, the absorbent material may be encapsulated in a capsule. The capsules may be substantially coated with a coating that allows it to pass through the gut and open in the intestine where the capsule may release the polymer to absorb fluid or specific ions that are concentrated in that particular position of the intestine. The individual particles or groups of particles may be encapsulated or alternatively, larger quantities of beads or particles may be encapsulated together.

In an exemplary method, the swelling rate of the polymer may be controlled by selecting particle or bead size, and or polymer with varied level of ion loading, to provide delivery of the polymer to specific locations in the gut before extensive swelling occurs. Larger sized particles have slower swelling rates. When given orally, the polymer may be used to supplement or replace dialysis treatments in dialysis patients, to supplement or replace diuretic therapy in patients with congestive heart failure, to supplement or replace diuretic and antihypertensive therapy in patients with hypertension and to supplement or replace these and dietary measures for treatment of fluid and/or sodium overload and/or potassium overload in patients with other diseases and syndromes, including those causing fluid retention in the body.

### Pharmaceutical Compositions

Pharmaceutical compositions are disclosed comprising a cross-linked polyelectrolyte polymer, including cross-linked polyelectrolyte polymeric beads, of the present disclosure. These compositions may be delivered to a subject, including a subject using a wide variety of routes or modes of administration. Preferred routes for administration are oral or intestinal.

A pharmaceutical composition or dosage form, including wherein the polymer is in admixture or mixture with one or more pharmaceutically acceptable carriers, excipients or diluents. Pharmaceutical compositions for use in accordance with the present disclosure may be formulated in conventional manner using one or more physiologically acceptable carriers compromising excipients and auxiliaries which facilitate processing of the polymer into preparations which may be used pharmaceutically. Proper formulation is dependent upon the route of administration chosen. Such compositions may contain a therapeutically effective amount of polymer and may include a pharmaceutically acceptable carrier. Pharmaceutically acceptable carriers include those approved by a regulatory agency of the Federal or a state government or listed in the U.S. Pharmacopeia or other generally recognized pharmacopeia for use in animals, and more particularly, in humans. Carriers can include an active ingredient in which the disclosed compositions are administered.

For oral administration, the disclosed compositions may be formulated readily by combining them with pharmaceutically acceptable carriers well known in the art. Such carriers enable the compositions of the disclosure to be formulated, preferably in capsules but alternatively in other dosage forms such as tablets, pills, dragees, capsules, liquids, gels, syrups, slurries, suspensions, wafers, sachets, powders, dissolving tablets and the like, for oral ingestion by a subject, including a subject to be treated. In some embodiments, the compositions or capsules containing the compositions, do not have an enteric coating.

The amount of the active cross-linked polyelectrolyte polymer, including cross-linked polyelectrolyte polymeric beads, are present in an effective amount, including, for example, in an amount effective to achieve therapeutic and/or prophylactic benefit. Effective doses may be extrapolated from dose-response curves derived from in vitro or animal model test systems. Dosage amount and interval may be adjusted individually to provide levels of cross-linked polyelectrolyte polymer, including cross-linked polyelectrolyte polymeric beads that are sufficient to maintain the desired therapeutic effect. The dosage regimen involved in a method of treatment may be determined by the attending physician, considering various factors which modify the action of polymer, e.g. the age, condition, body weight, sex and diet of the subject, the severity of disease, time of administration and other clinical factors.

The amount of compound administered will, of course, be dependent on the subject being treated, on the subject's weight, the nature and severity of the affliction, the manner of administration, and the judgment of the prescribing physician. The therapy may be repeated intermittently while symptoms are detectable or even when they are not detectable. The therapy may be provided alone or in combination with other agents.

The polyelectrolyte polymer of the present disclosure may be administered in combination with other therapeutic agents. The choice of therapeutic agents that may be co-administered with the compositions of the disclosure will depend, in part, on the condition being treated.

### EXAMPLES

### Example 1

This example demonstrates the preparation of an exemplary cross-linked polyelectrolyte polymer, such as a lightly crosslinked polyacrylic acid partially neutralized with sodium.

An inverse suspension process may be used with the following components: a monomer *(e.g.,* polyacrylic acid), solvent *(e.g.,* water), base for neutralization of monomer *(e.g.,* NaOH), lipophilic solvent *(e.g.,* Isopar L), suspending agent *(e.g.,* fumed silica such as Aerosil R972), chelating agent *(e.g.,* Versenex-80), polymerization initiator *(e.g.,* sodium persulfate), and cross-linking agent *(e.g.,* TMPTA). For example, cross-linked polyacrylate beads were prepared by adding eighty-eight kilograms acrylic acid and about eighty-seven kilograms of water to a suitable, agitated vessel and sparging air through the mixture. The mixture was continuously agitated and cooled while seventy-nine kilograms of 50% sodium hydroxide was added while the temperature of the mixture was advantageously maintained below about 40°C. In this manner about 80% neutralization of the acrylic acid was obtained. If desired, neutralization percentages of from about 60% to 100% were obtained by altering the amount of sodium hydroxide. Alternatively other basic sodium salts, such as sodium carbonate or sodium bicarbonate, are used in addition to basic salts of other alkali metals.

To a second, suitable, agitated reactor, about seven-hundred kilograms of Isopar L (or other lipophilic solvents such as toluene, heptane, cyclohexane) was added to 0.3 kilograms of fumed silica (Aerosil R972) that is pre-dispersed in about twenty kilograms of Isopar L (or other lipophilic solvent). Next, about 0.9 kilograms of Versenex-80 solution was added to the partially neutralized acrylic acid solution followed by the addition of 0.3 kilograms of trimethylolpropane triacrylate to the Isopar L / Aerosil R972 dispersion. About 0.06 kilograms sodium persulfate as a solution in about three kilograms of water was added to the partially neutralized acrylic acid solution. The partially neutralized acrylic acid solution may then be filtered.

The partially neutralized acrylic acid solution was transferred into the Isopar L in the second reactor. Optionally, the partially neutralized acrylic acid solution may be filtered at this point. The mixture was agitated for about fifteen to thirty minutes to achieve suspension of the aqueous monomer droplets while nitrogen (or other suitable inert gas) was sparged through the mixture during the agitation period. The reactor temperature may be increased to about 50°C at which point a second dispersion of Aerosil R972 (0.6 kilograms of Aerosil R972 in about twenty kilograms of Isopar L) may be added to the reaction mixture. Polymerization of the mixture was completed by heating the reaction mixture to about 65°C and holding the contents at about 65°C for about two to four hours after the peak exotherm was observed. The reactor contents were then cooled and placed under vacuum to remove water. About two-hundred and twenty kilograms of distillate was collected. The beads were isolated by centrifugation and dried under vacuum with a nitrogen bleed, if needed, at about 100°C.

The beads were screened to remove oversized agglomerates and fines. Typically, about one-hundred kilograms of cross-linked polyacrylate beads were obtained. If the residual acrylic acid level is too high, the cross-linked polyacrylate beads are reloaded to a suitable reactor containing Isopar L, water, and a small amount of sodium persulfate. After sparging the mixture with nitrogen, the beads were incubated at about 70 °C for about two to three hours. The mixture was then cooled and the cross-linked polyacrylate beads isolated, dried, and screened as before.

When the beads were screened, the mean particle size for the beads generally ranged from about 700 microns to about 1200 microns. The upper screen size ranged from 840 to 1400 microns (e.g., 24-16 mesh) and the lower screen size ranged from 540 to 840 microns (e.g., 36-24 mesh).

Optionally, the beads are placed into capsules (e.g., hard size 00 HPMC capsules). Such capsules are optionally coated. The following materials are used to prepare an exemplary coating suspension (% w/w): Eudragite L30D-55 (53.76%), Plasacryl (6.45%), triethyl citrate (2.58%) and sterile water (37.20%). For example, L30D-55 is dispensed into a steel container with agitation to create a vortex. Next, sterile water, Plasacryl and triehtyl citrate are added to the vortex. The capsules may then be sprayed with the mixture followed by drying.

### Example 2

This example demonstrates the preparation of substantially metal free cross-linked polyelectrolyte polymers, such as cross-linked polyacrylate polymer.

In an exemplary method, substantially metal free cross-linked polyacrylate were prepared by placing one-hundred and forty grams of glacial acrylic acid (not neutralized as in Example 1) into a three to five liter reactor with 2,200 to 2,500 milliliters of dilute acid, such as 1 M HCl, adding a water soluble cross linking agent such as 1,3-diglycerate diacrylate in a ratio chosen to produce the desired saline holding capacity *(e.g.,* 20-fold, 40-fold or more) with an initiator. After sparging the reactor with an inert gas, such as nitrogen, and agitating the reaction mixture to produce appropriately sized droplets, the reaction was started and allowed to proceed for two to four hours until substantially all of the monomer had reacted. The resultant mass of wet polymer was then be cut into smaller pieces *(e.g.,* one to two centimeter on a side), dried in a vacuum or in an inert atmosphere, and then disrupted *(e.g.,* by milling) to produce particles or powder.

### Example 3

The saline holding capacity of a cross-linked polyelectrolyte polymer, such as a cross-linked polyacrylate polymer, may be determined by known methods in the art.

In an exemplary method, saline holding capacity was determined with a 0.15 M sodium solution as follows. A pH seven buffer of sodium phosphate tribasic (Na₃PO₄•12H₂0; MW 380.124) was prepared by dissolving 19.0062 grams in about 950 milliliters pure water and adjusting the pH to a final pH of seven ± 0.1 with IN HCl before final dilution to one liter resulting in a solution with a sodium concentration of 0.15 M. Next, an amount of cross-linked polyelectrolyte, for example, cross-linked polyacrylate beads *(e.g.,* 0.1 ± 0.025 grams), were transferred to a tared filter tube and the mass of the beads recorded as in W1. Next, the tube was returned to the balance to record the weight of the tube plus the sample as W2. An excess *(e.g.,* more than seventy times the mass of polymer) amount of the pH 7.0 buffer *(e.g.,* ten milliliters) was then transferred to the tube containing the CLP sample. The tube was then placed on a flat bed shaker with shaking for two, four or six hours. When reduced sodium cross-linked polyacrylate polymer is tested for saline holding capacity, this time may be extended to twenty-four hours. After shaking, all excess fluid was removed from the tube *(e.g.,* no visible fluid in the tube). Last, the tube and sample were weighed and recorded as W3. The saline holding capacity (SHC) was calculated by dividing the mass of the dry cross-linked polyacrylate beads into the mass of the fluid absorbed, for example, SHC (g/g) = (W3-W2)/ (W1). According to the present disclosure, cross-linked polyelectrolyte polymeric beads, including polyacrylate beads prepared as described in Example 1, had a saline holding capacity of twenty grams per gram, forty grams per gram or more. Alternatively stated, such cross-linked polyelectrolyte polymeric beads, including where the polyelectrolyte is polyacrylate, can absorb 20-fold, 40-fold or more of their mass in a saline solution.

### Example 4

Cross-linked polyelectrolyte polymers, such as cross-linked polyacrylate polymers with varying counterion content *(e.g.,* counterions added, replaced, removed or reduced) may be prepared by any known method in the art.

A cross-linked polyelectrolyte polymer with a counterion content of 100% hydrogen was prepared *(e.g.,* 100% of total bound counterions are hydrogen). In an exemplary method, one-hundred grams of a cross-linked polyelectrolyte polymer, such as a partially neutralized cross-linked polyacrylate polymer *(e.g.,* prepared as described in Example 1 or 2 above) was placed into a vessel. Next, about 2,250 milliliters of pure *(e.g.,* trace metal or otherwise certified low metal) 1 M HCl was added to the vessel which is then stirred gently for two hours. The liquid was removed by decanting or filtration. If desired due to vessel size or for improved mass balance, the 2,250 milliliters of 1M HCl is divided into multiple batches and used sequentially. For instance, 750 milliliters were added, stirred with the polymer, and removed followed by two or more separate additions of 750 milliliters. The polymer was then rinsed with 2,250 milliliters of low metal content water to remove excess acid surrounding the polyelectrolyte such as a polyacrylate.

Alternatively, one-hundred grams of a cross-linked polyelectrolyte polymer, such as a cross-linked polyacrylate polymer were placed into a filtration funnel or a column equipped with a bottom filter. The polymer was then rinsed with about 2,250 milliliters of pure (e.g., trace metal or otherwise certified low metal) 1 M HCl for about an hour or more. Next, the polymer was rinsed with 2,250 milliliters of low metal content water.

### Example 5

Cross-linked polyelectrolyte polymers, such as cross-linked polyacrylate polymers with varying counterion content *(e.g.,* counterions added, replaced, removed or reduced) may be prepared by any known method in the art.

In an exemplary method to produce fully hydrogen loaded polymer, fifty liters of 1 N HCl was placed into a reactor with mixing between 100 and 1800 rpm, depending on the agitator and reactor. Next, four-thousand grams of a cross-linked polyelectrolyte polymer, such as a cross-linked polyacrylate polymer was added to the vessel. The cross-linked polyelectrolyte polymer was mixed for one hour and the acid then drained from the vessel. Thirty liters of 1 N HCl was poured into the vessel and the cross-linked polyelectrolyte mixed from 1500 to 1800 rpm for two hours. The mixer was then stopped and the acid solution drained from the vessel. Again, thirty liters of 1 N HCl was poured into the vessel and the cross-linked polyelectrolyte mixed from 100 to 1800 rpm for two hours. The mixer was then stopped and the acid solution drained from the vessel. Next, forty liters of sterile water was added to the vessel and the cross-linked polyelectrolyte polymer was mixed for one hour from 100 to 1800 rpm. After mixing, the water was drained from the vessel. Forty liters of sterile water was then added to the vessel with the cross-linked polyelectrolyte polymer. The cross-linked polyelectrolyte polymer was mixed for thirty minutes from 100 to 1800 rpm after which the water was drained from the vessel. Again, forty liters of sterile water was added to the vessel with the cross-linked polyelectrolyte polymer. The cross-linked polyelectrolyte polymer was mixed for thirty minutes from 1500 to 1800 rpm after which the water was drained from the vessel. Cross-linked polyelectrolyte polymer was then placed into a drying oven set to 95 °C ± 5 °C and dried for at least three hours, preferably under inert gas.

The resultant dried cross-linked polyacrylate with a counterion content of 100% hydrogen may be milled to produce cross-linked polyacrylate particles. In an exemplary method, a grinding apparatus (e.g., a COMIL^{®} apparatus) was loaded with the polyacrylate beads to just below the top of the impeller blade. The impeller was then turned on and set to 100% power. The grinding apparatus may be stopped every thirty minutes and allowed to cool for ten minutes before milling is resumed. Next, the milled material was poured through a sieving apparatus (e.g., a VORTI-SIV^{®} apparatus) set up with two screens (e.g., US Mesh # 35 and US Mesh # 70) to collect polyacrylate particles that were from 212 to 500 microns. Material greater than 500 microns was collected and again milled with the resulting particles again sieved for those particles between 212 to 500 microns. Milling and sieving may continue until the material greater than 500 microns no longer reduces in particle size. Particles less than 212 microns were collected through the grinding and sieving process as powder for use or may be discarded.

### Example 6

This example demonstrates the preparation of a 90% hydrogen, 10% sodium cross-linked polyelectrolyte polymer, such as a cross-linked polyacrylate polymer (e.g., 10% of the carboxylate sites are occupied by sodium and 90% of the carboxylate sites are occupied by hydrogen).

In an exemplary method, to obtain a 10% sodium, 90% hydrogen cross-linked polyelectrolyte, 5.2994 grams of sodium carbonate was dissolved in five-hundred milliliters water and added to 72.06 grams of acid-washed cross-linked polyelectrolyte polymer prepared as described in Example 5. The polymer was then dried in a vacuum oven or inert atmosphere oven until less than 5% moisture remains.

Potassium, other counterions or their mixtures may also be added to desired levels in a similar manner.

### Example 7

This example demonstrates the preparation of a cross-linked polyelectrolyte polymer, such as a cross-linked polyacrylate polymer with varying levels of bound sodium counterion (e.g., 5% sodium, 95% hydrogen; 20% sodium, 80% hydrogen; or 45% sodium, 55% hydrogen).

An x% sodium and (1-x)% hydrogen substituted polyelectrolyte polymer, such as a cross-linked polyacrylate polymer may be prepared from the 100% acid washed polymer (e.g., as described in Example 4 or 5 above) by adding G grams of the acid polymer (which has G/72.0627 moles carboxylic groups) to a solution of (x/100)(G/72.0627) moles of the desired couterion in the form of a suitable salt, mixing, removing undesirable salt product, if any was formed, by rinsing with pure water, and drying. Without wishing to be bound by a theory of the disclosure, it is believed that using carbonate, bicarbonate, hydroxide, or oxide salts generally avoids the need to rinse undesirable salts from the polymer.

For instance, a 5% sodium / 95% hydrogen substituted cross-linked polyelectrolyte polymer, such as a cross-linked polyacrylate polymer was prepared by adding 36.0313 grams of the fully acid loaded polymer to a dilute aqueous solution of (0.05)(36.0313/72.0627)(39.9971) = 0.9999 grams NaOH. The dilute solution was used to both prevent any damage to the polymer and to allow adequate fluid to allow fluid to enter every bead or particle of polymer rather than having only enough fluid to wet the polymer first added to the solution. After mixing, the suspension was placed into a vacuum oven with inert gas flushing and brought to less than 5% moisture content.

Similarly, a cross-linked polyelectrolyte polymer, such as a cross-linked polyacrylate polymer with 20% sodium / 80% hydrogen was made by adding 36.0313 grams of the fully acidified polymer to a dilute aqueous solution of (0.20)(36.0313/72.0627)(39.9971) = 3.9997 grams NaOH followed by adequate mixing and drying.

Likewise, a 45% sodium / 55% hydrogen cross-linked polyelectrolyte polymer, such as a cross-linked polyacrylate polymer was prepared by adding 36.0313 grams of the fully hydrogen polymer to a dilute solution of (.45)(36.0313/72.0627)(39.9971) = 8.9993 grams NaOH followed by adequate mixing and drying.

In an exemplary method, twelve grams of cross-linked polyelectrolyte polymer partially neutralized polyacrylate beads, prepared by reverse suspension polymerization of 80% neutralized acrylic acid with TMPTA as cross-linker, were placed into a fritted glass equipped column. Next, three hundred milliliters of 1 M HCl were passed through the column over a two hour time period. The beads were then rinsed with three-hundred milliliters of distilled water, removed from the column and dried overnight in a vacuum oven at 100°C. The resulting sodium free lightly cross-linked polyacrylic acid beads were then transferred into a beaker. Next, a solution of 7.09 grams of sodium carbonate in five-hundred milliliters of distilled water was added to the beaker. The solution was gently mixed and allowed to sit for several hours. The beads were then placed into a vacuum oven at 100°C overnight.

### Example 8

This example demonstrates the preparation of a cross-linked polyelectrolyte polymer, such as a cross-linked polyacrylate polymer with varying levels of bound potassium counterion (e.g., 25% potassium, 75% hydrogen; or 25% potassium, 25% choline, 50% hydrogen). Also demonstrated is a mixed cation polymer with potassium, hydrogen, and choline counterions.

For example, a 25% potassium, 75% hydrogen cross-linked polyelectrolyte polymer, such as a cross-linked polyacrylate polymer was prepared by adding 36.0313 grams of the fully acidified polymer (e.g., as described in Examples 4 or 5 above) to a dilute aqueous solution of (.25)(36.0313/72.0627)(56.1056) = 7.0132 grams KOH followed by adequate mixing and drying. Similarly, a 25% potassium, 25% choline, 50% hydrogen substituted cross-linked polyelectrolyte polymer, such as a cross-linked polyacrylate polymer was prepared by adding 36.0313 grams of the fully acidified polymer to an aqueous solution of 7.0132 grams KOH and 15.1473 grams choline hydroxide followed by adequate mixing and drying.

### Example 9

This example demonstrates the preparation of a cross-linked polyelectrolyte polymer, such as cross-linked polyacrylate polymer with varying levels of bound choline counterion (e.g., 50% choline, 50% hydrogen).

For example, a 50% choline / 50% hydrogen polymer was prepared by adding 36.0313 grams fully acid form polymer to a dilute aqueous solution of (.50)(36.0313/72.0627)(121.1781) = 30.2945 grams choline hydroxide followed by suitable mixing and drying.

### Example 10

Cross-linked polyelectrolyte polymeric beads such as cross-linked polyacrylate polymeric beads *(e.g.,* those produced by the methods described in Examples 6 and 7), may be used to remove fluid *in vivo.*

In an exemplary method, cross-linked polyacrylate with any level of bound sodium (e.g., 0% sodium, 100% hydrogen; 10% sodium, 90% hydrogen; 20% sodium, 80% hydrogen; 45% sodium, 55% hydrogen; or 80% sodium, 20% hydrogen) as prepared in Example 7 was used to remove fluid in rats. Three male Sprague Dawley rats were placed in each of four groups. Each group was fed regular PMI 5012 rat chow for six days while being housed in metabolic cages. Daily intake of food and water and daily output of feces and urine were recorded by weights. After six days, the groups were fed diets of PMI 5012 rat chow mixed with intact cross-linked polyelectrolyte polymeric beads to provide 3% of the diet as cross-linked polyelectrolyte polymeric beads. Four different sodium levels of cross-linked polyelectrolyte polymeric beads were used. The cross-linked polyelectrolyte polymeric beads has about 20% of the carboxylic sites bound to hydrogen and about 80% of the carboxylic acid sites bound to sodium *(e.g.,* prepared as described in Example 1). In addition, 55% hydrogen (45% sodium) cross-linked polyelectrolyte polymeric beads, 80% hydrogen (20% sodium), a 90% hydrogen (10% sodium) cross-linked polyelectrolyte polymeric bead, and a 100% hydrogen (0% sodium) cross-linked polyelectrolyte polymer were also used *(e.g.,* prepared as described in Example 2). The cross-linked polyelectrolyte polymeric beads were not absorbed through the intestine and absorbed liquid from the body. This liquid was excreted in the feces and caused an increased weight of fecal excretion. The last three days of the baseline period were compared with the last three days of the treatment period to allow determination of the equilibrated level of increase in fecal weight as shown in Table 2. For example, equilibrated baseline fecal weight was determined by averaging fecal weight from the groups over days 4, 5, and 6 (e.g., the last three days of the treatment period). Similarly, equilibrated treatment fecal weight was determined by averaging fecal weight form the groups over days 10, 11 and 12 *(e.g.,* the last three days of the treatment period). Subtraction of equilibrated fecal weight from baseline fecal weight resulted in a determination of the increase in fecal weight (Table 2).

**Table 2: Equilibrated Level of Increase in Fecal Weight**

| | **100% AW** | **90% AW** | **80% AW** | **55% AW** | **20% AW** |
|---|---|---|---|---|---|
| **Equilibrated Baseline Fecal Weight** | 6.02 | 5.73 | 7.27 | 6.37 | 6.04 |
| **Equilibrated Treatment Fecal Weight** | 8.79 | 8.73 | 10.52 | 10.81 | 9.86 |
| **Increase in Fecal Weight** | 2.77 | 3.00 | 3.25 | 4.44 | 3.82 |
| **P value treatment versus baseline** | 3.022 4E-06 | 6.8406 4E-06 | 9.752 E-05 | 2.9822E-06 | 4.62E-06 |
| **CLP dose (g)** | 0.42 | 0.42 | 0.61 | 0.54 | 0.58 |
| **Increased Feces per g CLP** | 6.65 | 7.10 | 5.30 | 8.29 | 6.63 |

Thus, each of the five different levels of sodium loading in CLP produced statistically significantly increases in fecal fluid excretion (p<<0.001). There were no statistically significant differences in the fecal fluid excretion per gram of cross-linked polyelectrolyte polymeric beads between any of the five different levels of sodium loading in cross-linked polyelectrolyte polymeric beads. This data suggests that acid-washed or low-sodium cross-linked polyelectrolyte polymeric beads remove fluid from mammals as well as cross-linked polyelectrolyte polymeric beads as it exists directly after preparation as the 20% hydrogen, 80% sodium crosslinked polyacrylate.

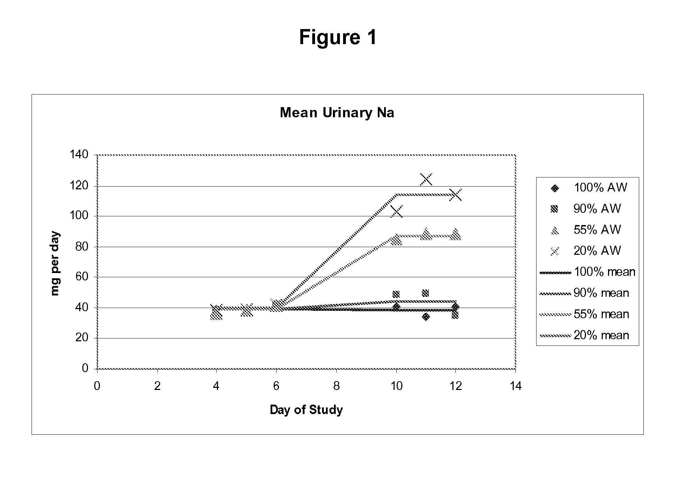

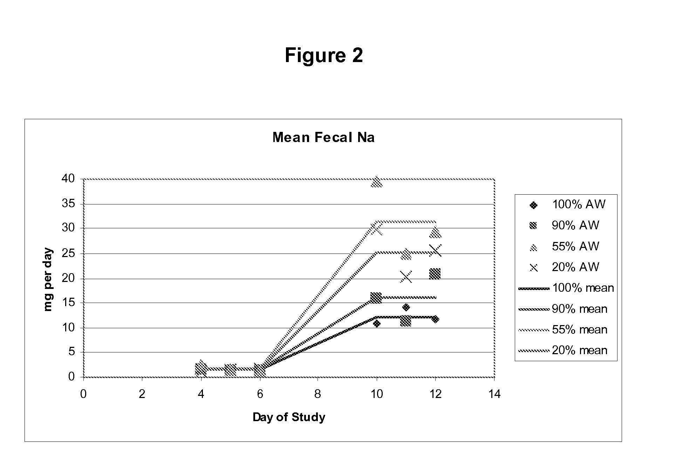

The sodium output (e.g., fecal and urinary) from these rats on the same days was also investigated as shown in Figures 1 and 2. Figure 1 shows urinary sodium output. The sodium changes in feces are shown in Figure 2. For the 20% acid (20% hydrogen, 80% sodium) CLP (X) the rats received an extra 120 mg of dietary sodium per day from the CLP with about 25 mg of this sodium excreted in the feces and about 80 mg in the urine. For the 100% acid form (100% hydrogen, 0% sodium) of CLP, no extra dietary sodium was given to the rats, but about an extra 10 mg of sodium over baseline was excreted in the feces with no change in the urinary sodium excretion.

### Example 11

This example demonstrates swelling of disrupted and intact CLP beads containing various amounts of sodium.

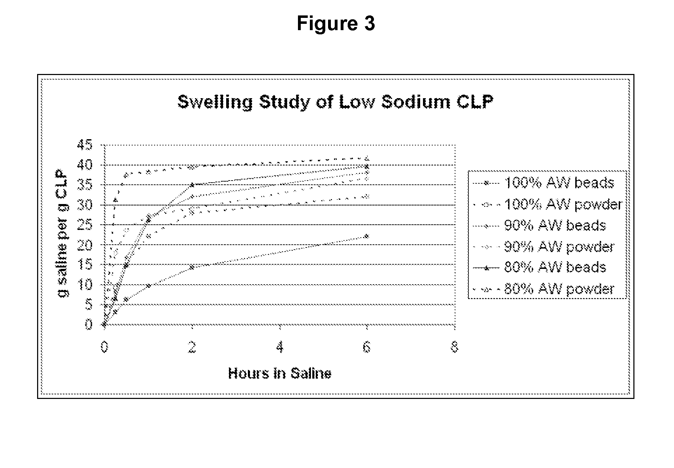

Intact and disrupted cross-linked polyelectrolyte polymeric beads containing 100% carboxylates as free acid (100% hydrogen), 90% carboxylates as free acid (90% hydrogen, 10% sodium), and 80% carboxylates in free acid form (80% hydrogen, 20% sodium) and their swelling in saline was determined. The results in Figure 3 demonstrate that all samples are superabsorbent polymers (e.g., absorbing more than twenty grams saline per gram of polymer) although absorption rates varied.

### Example 12

This example demonstrates the modulation (e.g., removal or donation) of ions (e.g., sodium and potassium) and/or removal of fluid (e.g., saline) in rats administered a cross-linked polyelectrolyte polymer (CLP) with varying levels of bound counterions.

In an exemplary method, nine Sprague Dawley rats were placed after acclimatization, into individual metabolic cages and fed pulverized PMI 5012 rodent chow (1010 milligrams calcium, 1080 milligrams potassium, 210 milligrams magnesium, and 280 milligrams sodium per one-hundred grams chow). Rats were randomized into three groups to receive various forms of cross-linked polyelectrolyte polymer ("CLP"). The CLP was prepared as described in Example 1 and the counterion content was varied as described in Examples 6 and 7. Each rat served as its own control, with a baseline period on the control diet and then a treatment period with CLP included in the diet. Rats were fed PMI 5012 rat chow for six days while being housed in metabolic cages. On the sixth day, each of five randomized groups of rats, have their diet replaced with CLP beads bound to 100%, 90%, 80%, 55% or 20% hydrogen. Remaining sites on the CLP were substituted with sodium (e.g., for CLP bound to 80% hydrogen, 20% of the sites are bound to sodium). Daily intake of food and water and daily output of feces and urine were recorded by weight. Fecal weight, fecal sodium and fecal potassium levels were calculated on days four, five, six, ten, eleven and twelve. Levels obtained on days four, five and six are compared to those obtained on days ten, eleven and twelve, respectively for each of the rat groups. For example, an amount of fluid removed was determined by subtracting the fecal weight on day ten by the fecal weight on day four. Similarly, the level of fecal sodium on day four was compared to the level of fecal sodium on day ten. Differences in fecal weight, fecal sodium and fecal potassium from the three comparisons from the three groups of rats were determined and mean and standard deviation are calculated (Table 3). The data in Table 3 are another representation of the data obtained in the rat experiment described in Example 10.

For all groups, there was no statistically significant differences in the amount of fluid removed and the type of CLP used (e.g., 100% acid wash CLP removes just as much fluid as the 20% acid form of CLP). Urine and fecal sodium increased in relation to the amount of sodium in the CLP administered (*e.g*., 100% acid (no sodium) had less increase that 20% acid (80% sodium). Urinary levels of sodium also increased suggesting in relation to the amount of sodium on the CLP suggesting that at least some of the sodium may be made available to the body (e.g., released or donated) and be excreted in the urine. In rats administered 100% H-CLP, sodium was removed from the rat (e.g., 10.74 mg) (Table 3). Conversely, administration of 55% or 20% H-CLP resulted in donation of sodium to the rat. However, 90%-80% H-CLP resulted in a near net zero change in sodium level (Table 3). Urine potassium appeared to increase for all treatment groups but was only significant for 55% acid washed CLP (p<0.01). Fecal potassium increased significant (p<0.01) for all treatments (Table 3). No statistically significant changes were observed in magnesium and calcium levels.

To determine the impact on increasing the dose of CLP, the experiment as described above was conducted using a dose titration of 100% H-CLP as three, four, five, six or ten percent of total diet in rat. As expected, increasing the CLP in the diet led to an increased amount of fluid removal. However, when H-CLP reached 10% of the diet, rats decreased their intake of food possibly due to taste or texture of CLP. Accordingly, the decreased food intake affected the total amount of CLP consumed such that it is unlikely that they received the full intended study dose (Table 4).

Additionally, a dose titration was performed to access the impact of raising the percent of 20% H, 80% Na -CLP in the rat diet. Methods were followed as described above with the exception that CLP beads used were as follows: 20% H, 80% Na. The CLP consisted of three, six and ten percent of the rat diet. As expected, an increase in the percentage of CLP in the total diet increased both fecal and urine sodium levels (e.g., from three to six percent of the diet) (Table 5). As discussed above, when the CLP reached 10% of the rat diet it was unlikely that rats consumed the intended dose.

**Table 3: Various Forms of H-CLP as 3% of Total Diet**

| **Change in % Na on CLP** | | | | | | | | | | |
|---|---|---|---|---|---|---|---|---|---|---|
| **CLP Composition** | **Mean Fluid** | **SD Fluid** | **Mean Na** | **SD Na** | **Mean K** | **SDK** | **Na Removed (mean)** | **Na Removed (SD)** | **K Removed (mean)** | **K Removed (SD)** |
| 100% H-CLP as 3% of diet | 2.77 | 1.30 | 10.74 | 3.03 | 7.25 | 2.13 | 10.80 | 3.16 | | |
| 95% H-CLP as 3% of diet | 5.03 | 1.54 | 32.70 | 14.91 | 22.75 | 11.45 | 26.84 | 14.15 | | |
| 90% H-CLP as 3% of diet | 3.00 | 1.19 | 15.78 | 6.65 | 8.49 | 5.77 | 5.60 | 7.25 | | |
| 80% H-CLP as 3% of diet | 3.25 | 1.04 | 23.94 | 4.72 | 8.58 | 4.63 | 6.06 | 4.25 | | |
| 55% H-CLP as 3% of diet | 4.44 | 1.53 | 29.47 | 9.87 | 9.10 | 3.29 | -29.55 | 9.32 | | |
| 20% H-CLP as 3% of diet | 3.82 | 1.50 | 25.77 | 8.94 | 8.29 | 4.26 | -89.75 | 12.31 | | |

**Table 4: Dose Titration of 100% H-CLP as Percentage of Total Diet**

| **Dose of H-CLP** | | | | | | | | | | |
|---|---|---|---|---|---|---|---|---|---|---|
| **CLP Composition** | **Mean Fluid** | **SD Fluid** | **Mean Na** | **SD Na** | **Mean K** | **SD K** | **Na Removed (mean)** | **Na Removed (SD)** | **K Removed (mean)** | **K Removed (SD)** |
| 100% H-CLP as 3% of diet | 2.77 | 1.30 | 10.74 | 3.03 | 7.25 | 2.13 | 10.80 | 3.16 | | |
| 100% H-CLP as 3% of diet | 0.77 | 1.48 | 12.53 | 4.08 | 6.35 | 4.43 | | | | |
| 100% H-CLP as 4% of diet | 0.33 | 2.26 | 5.83 | 4.62 | 3.16 | 5.57 | | | | |
| 100% H-CLP as 5% of diet | 9.23 | 5.31 | 47.48 | 24.01 | 90.17 | 16.58 | | | | |
| 100% H-CLP as 6% of diet | 3.93 | 2.34 | 29.51 | 6.19 | 63.25 | 5.57 | | | | |
| 100% H-CLP as 10% of diet | 1.42 | 2.51 | 17.68 | 9.26 | 41.49 | 13.02 | | | | |

**Table 5: Dose Titration of 20% H 80% Na-CLP as Percentage of Total Diet**

| **Dose of 80% Na-CLP** | | | | | | | | | | |
|---|---|---|---|---|---|---|---|---|---|---|
| **CLP Composition** | **Mean Fluid** | **SD Fluid** | **Mean Na** | **SD Na** | **Mean K** | **SD K** | **Na Removed (mean)** | **Na Removed (SD)** | **K Removed (mean)** | **K Removed (SD)** |
| 20% H 80% Na CLP as 3% of diet | 0.77 | 1.81 | 12.26 | 3.61 | 5.48 | 4.97 | -95.42 | 3.46 | | |
| 20% H 80% Na CLP as 6% of diet | 6.61 | 2.64 | 36.78 | 8.70 | 36.99 | 14.70 | -142.24 | 13.39 | | |
| 20% H 80% Na CLP as 10% of diet | 13.67 | 4.18 | 38.81 | 13.37 | 135.29 | 28.68 | -302.78 | 16.66 | | |

### Example 13

This example demonstrates the modulation (e.g., removal or donation) of ions (e.g., sodium and potassium) and/or removal of fluid (e.g., saline) in rats administered a cross-linked polyelectrolyte polymer (CLP) with varying levels of bound counterions. The CLP was prepared as described in Example 1 and the counterion content was varied as described in Examples 6 and 7.

Methods are followed as described in Example 7 with the exception that the CLP beads used were as follows: 100% H; 75% H, 25% K; or 50% H, 50% K.

For all groups, urine and fecal potassium increased in relation to the amount of sodium in the CLP administered (*e.g*., 100% acid (no potassium) had less increase than 50% acid (50% sodium) (Table 6). Urine and fecal potassium increased in relation to the amount of sodium in the CLP administered (*e.g*., 100% acid (no potassium) had less increase that 50% acid (50% potassium) (Table 6). Urinary levels of potassium also increased suggesting in relation to the amount of potassium on the CLP suggesting that at least some of the potassium may be made available to body and be excreted in the urine. No statistically significant changes were observed in magnesium and calcium levels. There was no statistically significant differences in the amount of fluid removed with the type of CLP used (e.g., 100% acid wash CLP removes just as much fluid as the 50% acid washed 50% potassium form of CLP) (Table 6).

**Table 6: Dose Titration of 100% H-CLP as Percentage of Total Diet**

| **Change in % K on CLP** | | | | | | | | | | |
|---|---|---|---|---|---|---|---|---|---|---|
| **CLP Composition** | **Mean Fluid** | **SD Fluid** | **Mean Na** | **SD Na** | **Mean K** | **SD K** | **Na Removed (mean)** | **Na Removed (SD)** | **K Removed (mean)** | **K Removed (SD)** |
| 100% H-CLP as 5% of diet | 9.23 | 5.31 | 47.48 | 24.01 | 90.17 | 16.58 | | | 90.17 | 16.58 |
| 75% H 25 % K-CLP as 5% of diet | 13.78 | 1.81 | 44.80 | 8.74 | 127.30 | 11.01 | | | 37.35 | 9.73 |
| 50% H 50% K-CLP as 5% of diet | 14.84 | 2.44 | 43.24 | 7.11 | 153.73 | 15.48 | | | -33.16 | 14.18 |

### Example 14

This example demonstrates the modulation (e.g., removal or donation) of ions (e.g., sodium and potassium) and/or removal of fluid (e.g., saline) in rats administered a cross-linked polyelectrolyte polymer (CLP) with varying levels of bound counterions.

Methods are followed as described in Example 7 with the exception that CLP beads used were as follows: 100% H-CLP as 5% of diet; 50% Choline CLP as 5% of diet; 100% Choline CLP as 5% of diet; 100% L-Lysine CLP as 5% of diet. Additionally, a second experiment was conducted as described in Example 7 with the exception that the CLP beads used and doses administered were as follows: 100% H-CLP as 5% of diet; 100% NH4 CLP as 5% of diet; 50% NH4 CLP as 10% of diet; 100% NH4 CLP as 10% of diet.

Administration of 50% Choline CLP as 5% of diet resulted in similar fluid removal as 100% H-CLP (Table 7). Notably, sodium and potassium removal with 50% Choline CLP as 5% of diet was less than 100% H-CLP suggesting that choline may be used to substitute hydrogen without adversely affecting body sodium and potassium levels. Similarly, 100% NH4 CLP removed a similar amount of fluid as the 100% H CLP while removing a significantly reduced amount of sodium and potassium (Table 8). No statistically significant changes were observed in magnesium and calcium levels.

**Table 7: Dose Titration of 100% H-CLP as Percentage of Total Diet**

| **Organic Cations** | | | | | | | | | | |
|---|---|---|---|---|---|---|---|---|---|---|
| **CLP Composition** | **Mean Fluid** | **SD Fluid** | **Mean Na** | **SD Na** | **Mean K** | **SD K** | **Na Removed (mean)** | **Na Removed (SD)** | **K Removed (mean)** | **K Removed (SD)** |
| 100% H-CLP as 5% of diet | 9.23 | 5.31 | 47.48 | 24.01 | 90.17 | 16.58 | | | | |
| 50% Choline CLP as 5% of diet | 8.71 | 3.99 | 10.37 | 3.05 | 18.31 | 9.80 | | | | |
| 100% Choline CLP as 5% of diet | 1.84 | 3.23 | 1.57 | 0.99 | 2.05 | 2.83 | | | | |
| 100% L-Lysine CLP as 5% of diet | 5.71 | 3.19 | 13.52 | 7.74 | 4.41 | 3.22 | | | | |

**Table 8: Dose Titration of 100% H-CLP as Percentage of Total Diet**

| **Ammonium-CLP Dose Response** | | | | | | | | | | |
|---|---|---|---|---|---|---|---|---|---|---|
| **CLP Composition** | **Mean Fluid** | **SD Fluid** | **Mean Na** | **SD Na** | **Mean K** | **SD K** | **Na Removed (mean)** | **Na Removed (SD)** | **K Removed (mean)** | **K Removed (SD)** |
| 100% H-CLP as 5% of diet | 9.23 | 5.31 | 47.48 | 24.01 | 90.17 | 16.58 | | | | |
| 100% NH4 CLP as 5% of diet | 7.82 | 2.18 | 45.25 | 9.11 | 35.25 | 7.11 | | | | |
| 50% NH4 CLP as 10% of diet | 1.47 | 3.89 | 14.37 | 5.51 | 52.56 | 36.37 | | | | |
| 100% NH4 CLP as 10% of diet | 3.19 | 2.29 | 30.65 | 8.75 | 59.61 | 15.85 | | | | |

### Example 15

This example demonstrates the modulation (e.g., removal or donation) of ions (e.g., sodium and potassium) and/or removal of fluid (e.g., saline) in rats administered a cross-linked polyelectrolyte polymer (CLP) with varying levels of bound counterions.

Methods are followed as described in Example 7 with the exception that CLP beads used and doses administered were as follows: 20% H, 80% Na CLP as 3% of diet; 20% H, 80% Na CLP as 6% of diet; or 20% H, 80% Na CLP as 10% of diet.

Administration of CLP as a higher percentage of the rat diet increased the amount of fluid removed (e.g., administration of 20% H 80% Na CLP as 6% of the diet removed more fluid than 20% H 80% Na CLP as 3% of the diet) (Table 9). Also, the amount of sodium donated to the rat increased as the dosage increased as indicated by fecal and urine sodium levels. (Table 9). No statistically significant changes were observed in magnesium and calcium levels.

**Table 9: Dose Titration of 100% H-CLP as Percentage of Total Diet**

| **Dose of 80% Na-CLP** | | | | | | | | | | |
|---|---|---|---|---|---|---|---|---|---|---|
| **CLP Composition** | **Mean Fluid** | **SD Fluid** | **Mean Na** | **SD Na** | **Mean K** | **SD K** | **Na Removed (mean)** | **Na Removed (SD)** | **K Removed (mean)** | **K Removed (SD)** |
| 20% H 80% Na CLP as 3% of diet | 0.77 | 1.81 | 12.26 | 3.61 | 5.48 | 4.97 | -95.42 | 3.46 | | |
| 20% H 80% Na CLP as 6% of diet | 6.61 | 2.64 | 36.78 | 8.70 | 36.99 | 14.70 | -142.24 | 13.39 | | |
| 20% H 80% Na CLP as 10% of diet | 13.67 | 4.18 | 38.81 | 13.37 | 135.29 | 28.68 | -302.78 | 16.66 | | |

### Example 16

A clinical trial of an exemplary CLP was conducted. Objectives of the clinical trial included: to determine the amount of fluid absorbed per gram of CLP administered as assessed by stool weight compared with baseline period in a fasted state; to determine if the addition of mannitol, an osmotic agent, enhances stool weight increase compared with baseline period and with CLP administered alone; and to determine the effect of the capsule enteric-coating on removal of calcium, magnesium, and potassium.

Primary endpoints included: net sodium balance compared among treated and control groups. Secondary endpoints included: change in stool weight compared among treated and control groups; net balance of calcium, magnesium, potassium, iron, copper, zinc and phosphorous compared among treated and control groups; fluid consumed and excreted in the treated groups compared with the control group; and safety and tolerability based upon review of vital signs, clinical safety labs and adverse events.

An open-label, non-randomized, multiple-dose study in six healthy subjects divided into two groups of three subjects in each group was conducted. Subjects participated in a six day baseline period during which diet was controlled and stool weight and fluid balance was determined. The treatment period began on day seven. During the treatment period, dosing with CLP capsules took place, diet was controlled and stool weight and fluid balance was determined. The subjects selected had the ability to swallow up to 27 capsules each day of the study that included CLP dosing.

Subjects were placed into one of two groups on day six based upon the previous five days of stool weights, so that the average daily stool weight is approximately equal across groups. Group 1 received CLP in enteric-coated capsules. Group 2 received CLP in enteric-coated capsules mixed with mannitol. CLP with and without mannitol was supplied in hard size 00 HPMC enteric-coated capsules and sent as a finished dosage.

The encapsulated CLP used in the study incorporates an enteric coating that is reported to dissolve at approximately pH 5.5 while being insoluble at more acidic pH values. Gastric pH is usually below pH 2 while the stomach is empty, rises to about pH 7 with the ingestion of food, and falls back to about pH 2 within ten to fifteen minutes of the start of a meal as gastrointestinal hormones are secreted, causing the secretion of large amounts of hydrochloric acid. In the duodenum a higher pH is encountered and the coating dissolves. Since passage through the duodenum takes about five minutes, the CLP is expected to be exposed to the intestinal fluid first in the upper jejunum and absorb 90% of its fluid capacity during the ninety minutes of small bowel transit.

During the Treatment Period, oral total daily doses of ten grams CLP was divided into four doses and administered for six days, for a total of twenty-four consecutive doses in the treatment period. For Group I, the CLP dose was milled with 0.73 grams per capsule and divided into four doses in capsules. For Group 2, the 10 gram CLP is milled with 0.365 grams per capsule and with 10 grams mannitol in the same capsule (0.365 grams per capsule) and divided in four doses in capsules.

A standardized diet was administered throughout the study, including a six-day baseline period during which no CLP dosing occurs. All meals provided to the subjects are controlled for the number of calories, fat and fiber content. CLP was administered in a fasted state, at least one hour prior to each of four meals/snacks (e.g., breakfast, lunch, dinner and snack) and administered with water. Subjects were restricted from additional fluids for one hour pre and post dose.

All urine was collected separately for each twenty-four hour period, measured, and then discarded except for study day five and study day eleven when the urine for the entire day was pooled to allow an aliquot to be sent for potassium, magnesium and calcium analysis.

All feces were collected and each sample was individually weighed and the color and consistency of the stool noted. The weights were added together to determine the total fecal weight resulting from each day's intake. The entire weight of stool between the color markers was also totaled separately for both the baseline period and the experimental period.

All feces eliminated after consumption of the first controlled meal were collected as individual samples in tared collection containers, labeled, accurately weighed, then frozen and stored at or below -20°C. Individual stools during each twenty-four hour interval were collected and stored separately. Protocol number, subject number, study day, date and time of sample collection, and weight of sample and container was clearly and permanently indicated on collection containers for each individual stool. Sample time, presence of beads, color, consistency and weight were also recorded in the medical record. Fecal and urine collections from days five and eleven were submitted for analysis of calcium, magnesium, and potassium.

No formal statistical analysis of fecal weights were performed. Fecal weight data was summarized using descriptive statistics (mean, standard deviation, median, minimum and maximum) as appropriate. The cumulative weight of all stool samples excreted during the Baseline Period was compared to the cumulative weight of the stool samples excreted during the Treatment Period, by calculating change from baseline values for each subject. Comparisons were made both within and among the two treatment groups.

Summary statistics for fecal and urine concentrations of calcium, magnesium, and potassium on day five and day eleven (as well as for changes from Day 5 to Day 11) were presented by treatment group. Fluid balance data was summarized using descriptive statistics (e.g., mean, standard deviation, median, minimum and maximum) as appropriate. Fluid intake and output were measured for each twenty-four hour period during the study. These measurements were compared between baseline and treatment periods.

Change in fecal metal excretion (e.g., sodium, potassium, magnesium and calcium) for varying administrations of CLP are shown in Table 10.

**Table 10: Change in Fecal Metal Excretion (mg/day)**

| **CLP** | **Na** | **K** | **Mg** | **Ca** |
|---|---|---|---|---|
| Milled, uncoated | 1279 | 82 | 1768 | 1245 |
| Milled, uncoated; mannitol | 803 | -8 | 448 | 369 |
| Beads, uncoated; mannitol | 832 | 64 | 715 | 843 |
| Milled, coated pH 5.5 | 1445 | 119 | 1133 | 1518 |
| Milled, coated pH 5.5; mannitol | 1469 | 45 | 579 | 1335 |

### Example 17

A clinical trial of an exemplary CLP was conducted. Objectives of the clinical trial included: to determine the safety, tolerability and efficacy of the CLP on removal of sodium, calcium, magnesium, potassium, iron, copper, zinc and phosphorous; to determine the amount of fluid absorbed per gram of CLP administered as assessed by stool weight compared among treated and control groups.

Primary endpoints included: net sodium balance compared among treated and control groups. Secondary endpoints included: change in stool weight compared among treated and control groups; net balance of calcium, magnesium, potassium, iron, copper, zinc and phosphorous compared among treated and control groups; fluid consumed and excreted in the treated groups compared with the control group; and safety and tolerability based upon review of vital signs, clinical safety labs and adverse events.

For this clinical trial, an open-label, randomized, multiple-dose escalation study was performed in up to thirty-five healthy subjects divided into five groups. Another five subjects in the first group served as a control group. Up to five additional subjects may be added as a subsequent control group. For Group I, the CLP dose was 15 grams (milled with 0.75 grams per capsule) divided into four doses in capsules. For Group 2, the CLP dose was 25 grams (milled with 0.75 grams per capsule) divided in four doses in capsules. For Group 3, the CLP dose was 35 grams (milled with 0.75 grams per capsule) divided in four doses in capsules. For Group 4, the CLP dose was 45 grams (milled with 0.75 grams per capsule) divided in four doses in capsules. For Group 5, the CLP dose was 55 grams (milled with 0.75 grams per capsule) divided in four doses in capsules.

The initial dose of CLP was fifteen grams per day and given four times daily. The dose may be increased by increments of ten grams per day in subsequent groups. Based on the extent of sodium removal seen at fifteen grams per day it may be decided to reduce the dose of CLP rather than increase it.

During the nine day treatment period, dosing with CLP capsules took place. Diet was controlled with all participants having the identical meals. Stool weight, electrolyte and fluid balance was determined.

For the first dosing group, each day all meals and snacks representing one subject was homogenized and its sodium, potassium, calcium, phosphorus, iron, copper, zinc and magnesium content determined. All meals provided to the subjects were controlled for the number of calories, level of sodium (∼5000 mg per day +/- 100 mg), fiber content (10-15 g per day), fat content and approximate recommended Dietary Reference Intakes.

CLP was administered immediately prior to each of four meals/snacks and administered with water. Subjects remained in the clinical research unit for the duration of the study. The subjects were requested to consume all of their meals. Meals that are not fully consumed were collected for an entire twenty-four hour period, weighed and frozen for possible metal analysis.

All urine was collected separately for each twenty-four hour period, measured, and then pooled for the entire day to allow an aliquot to be sent for sodium, potassium, calcium and magnesium analysis. However, prior to pooling the afternoon specimen, the urine pH and osmolality were determined on a two milliliter aliquot to be discarded.

All feces were collected and each sample was individually weighed and the color and consistency of the stool noted. The weights were added together to determine the total fecal weight resulting from each day's intake. The twenty four hour period for both stool and urine collection was considered from midnight to midnight.

For each dose level, oral doses of CLP were divided into four doses and administered for nine days, for a total of thirty-six consecutive doses. Doses were given within ten minutes of the scheduled time for each subject.

Subjects were required to fast for at least eight hours at screening and four hours at admission prior to the collection of blood and urine samples for clinical laboratory tests. Fasting was not required prior to urine and blood samples taken during the study. Water ad libitum was allowed during the periods of fasting.

All feces eliminated after consumption of the first controlled meal were collected as individual samples in tared collection containers, labeled, accurately weighed, then frozen and stored at or below -20°C. Individual stools during each twenty four hour interval were collected and stored separately. Fecal collections were submitted for analysis of sodium, calcium, magnesium, potassium, iron, copper, zinc and phosphorus.

The urine was pooled for each day and analyzed for sodium, potassium, magnesium, calcium and phosphorous. A two milliliter aliquot from an afternoon specimen on Days 1 through 9 was sent for analysis of pH and osmolality. On Day nine, oxalate was also analyzed.

Daily fecal weight data was summarized by treatment group using descriptive statistics (mean, standard deviation, median, minimum and maximum) as appropriate. Summary statistics were presented by treatment group for the cumulative weight of all stool samples excreted on Days 1-9 and Days 4-9. If a "steady-state" period was identified, summary statistics for cumulative fecal weights were presented by treatment group for this period as well. Informal comparisons were made among the groups.

Summary statistics for daily fecal and urine content and concentrations of sodium, calcium, magnesium, potassium and phosphorus (plus copper, iron and zinc only in the stool) were presented by treatment group. Summary statistics may also be presented by treatment group for cumulative concentrations during the study (e.g., Days 1-9, Days 4-9). The urine osmolality and pH taken daily and oxalate on Day 9 was summarized by treatment group. Additionally, treatment group mean daily values of sodium, calcium, magnesium, potassium, phosphorus, iron, copper and zinc may be plotted versus study day.

Fluid balance data was summarized using descriptive statistics (mean, standard deviation, median, minimum and maximum) as appropriate. Fluid intake and output were measured for each twenty-four hour period during the study. These measurements were summarized and compared among treatment groups. The net balance of sodium, magnesium, calcium, potassium and phosphorus was calculated based on the analysis of diet, urine and stool samples. These net balance data were summarized by treatment group using descriptive statistics.

Changes in fecal metal excretion (e.g., sodium, potassium, magnesium and calcium) for varying doses of CLP are shown in Tables 11-14.

**Table 11: Change in Fecal Metal Excretion (mg/day), 0 grams CLP**

| **Day** | **Na** | **K** | **Mg** | **Ca** |
|---|---|---|---|---|
| 1 | 33.47 | 906.48 | 141.18 | 554.89 |
| 2 | 70.50 | 239.64 | 342.08 | 1663.39 |
| 3 | 12.09 | 728.73 | 112.11 | 691.18 |
| 4 | 114.82 | 394.42 | 292.65 | 2005.64 |
| 5 | 21.54 | 453.28 | 149.08 | 1134.07 |
| 6 | 32.82 | 680.21 | 182.25 | 1351.66 |
| 7 | 151.47 | 289.44 | 289.25 | 2003.07 |
| 8 | 44.87 | 259.00 | 120.19 | 1059.03 |
| 9 | 45.51 | 0.00 | 108.96 | 866.05 |

**Table 12: Change in Fecal Metal Excretion (mg/day), 7.5 grams CLP**

| | **Na** | **K** | **Mg** | **Ca** |
|---|---|---|---|---|
| 1 | 55.93 | 593.73 | 271.46 | 1297.63 |
| 2 | 133.18 | 1053.57 | 324.59 | 1810.54 |
| 3 | 360.72 | 1430.32 | 238.99 | 1442.42 |
| 4 | 587.86 | 2282.63 | 274.82 | 1874.84 |
| 5 | 383.67 | 1376.12 | 151.25 | 1062.92 |
| 6 | 398.14 | 1635.60 | 209.58 | 1456.87 |
| 7 | 683.07 | 1903.55 | 266.89 | 1557.50 |
| 8 | 569.40 | 2052.82 | 279.79 | 1787.31 |
| 9 | 343.50 | 1363.89 | 181.56 | 113.91 |

**Table 13: Change in Fecal Metal Excretion (mg/day), 15 grams CLP**

| | **Na** | **K** | **Mg** | **Ca** |
|---|---|---|---|---|
| 1 | 21.97 | 573.79 | 238.08 | 996.26 |
| 2 | 105.53 | 1108.00 | 230.08 | 960.19 |
| 3 | 344.97 | 1696.68 | 189.78 | 989.96 |
| 4 | 587.97 | 1831.73 | 164.50 | 915.86 |
| 5 | 1056.03 | 3028.69 | 264.60 | 1585.19 |
| 6 | 1151.75 | 2189.71 | 227.81 | 1279.78 |
| 7 | 1037.25 | 2012.49 | 227.34 | 1262.60 |
| 8 | 1113.89 | 1814.38 | 214.33 | 1300.79 |
| 9 | 1043.76 | 2154.11 | 221.97 | 1269.37 |

**Table 14: Change in Fecal Metal Excretion (mg/day), 25 grams CLP**

| | **Na** | **K** | **Mg** | **Ca** |
|---|---|---|---|---|
| 1 | 120.38 | 583.05 | 221.52 | 1025.54 |
| 2 | 850.29 | 1254,19 | 200.11 | 970.26 |
| 3 | 735.65 | 1557.08 | 125.49 | 731.16 |
| 4 | 2016.94 | 3684.93 | 254.31 | 1411.68 |
| 5 | 1790.12 | 4136.99 | 230.05 | 1354.08 |
| 6 | 1912.24 | 5120.06 | 263.19 | 1593.74 |
| 7 | 2518.92 | 5259.13 | 3216.06 | 1760.23 |
| 8 | 2538.84 | 3857.10 | 293.36 | 1520.50 |
| 9 | 1650.54 | 2446.57 | 174.06 | 899.62 |

### Example 18

A clinical trial of an exemplary CLP was conducted. Objectives of the clinical trial included: to determine the amount of fluid absorbed per gram of CLP administered as assessed by stool weight compared with baseline period in both a fed and fasted state; to determine if the addition of mannitol, an osmotic agent, enhances stool weight increase compared with baseline period and with CLP administered alone; and to determine the impact of CLP on the potential removal of trace elements such as Ca, Mg and K.

Primary endpoints included: net sodium balance compared among treated and control groups. Secondary endpoints included: change in stool weight compared among treated and control groups; net balance of calcium, magnesium, potassium, iron, copper, zinc and phosphorous compared among treated and control groups; fluid consumed and excreted in the treated groups compared with the control group; and safety and tolerability based upon review of vital signs, clinical safety labs and adverse events.

For this clinical trial, an open-label, non-randomized, multiple-dose study in eighteen healthy subjects divided into six groups of three subjects in each group was conducted. Subjects were placed into one of six groups on day six, based upon the previous five days of stool weights, so that the average daily stool weight is approximately equal across groups. A summary of treatment groups is indicated in Table 15.

**Table 15: Treatment Groups**

| **Group** | **CLP Details** | **Mannitol** | **Fasted/Fed** |
|---|---|---|---|
| 1 | milled, 212µ - 500µ particles | No | Fasted |
| 2 | milled, 212µ - 500µ particles | Yes, with CLP | Fasted |
| 3 | 500µ - 710µ beads | Yes, separate uncoated capsules | Fasted |
| 4 | 710µ - 1000µ beads | Yes, separate uncoated capsules | Fasted |
| 5 | milled, 212µ - 500µ particles | No | Fed |
| 6 | milled, 212µ - 500µ particles | Yes, with CLP | Fed |

CLP (milled) with and without mannitol (Groups 1, 2, 5 and 6) was in size 00 HPMC capsules that are uncoated. CLP (beads) and mannitol alone were supplied in bottles so that they may put into size 00 HPMC capsules (Groups 3 and 4).

CLP was administered according to treatment assignment (Groups 1, 2, 3, and 4) in a fasted state, at least one hour prior to each of four meals/snack and administered with water. Subjects were restricted from additional fluids for one hour pre and post dose. For Groups 5 and 6, CLP was administered in a fed state, thirty minutes following the end of a meal and evening snack, administered with water and without any fluid restriction.

All meals provided to the subjects are controlled for the number of calories, fat and fiber content; and the same meals was provided on corresponding days of Baseline and Treatment periods.

All urine was collected separately, weighed, and then discarded except for Study Day 5 and Study Day 11 when the urine for the entire day was pooled to allow an aliquot to be sent for K, Mg, and Ca analysis. All feces were collected in separate containers.

Each sample was individually weighed and the color and consistency of the stool noted. The weights were added together to determine the total fecal weight resulting from each day's intake. The entire weight of stool between the color markers are also totaled separately for both the baseline period and the experimental period. Stools were also examined for the presence of beads during the treatment period.

During the Treatment Period, oral total daily doses of ten grams CLP was divided into four doses and administered with water for six days, for a total of twenty-four consecutive doses in the treatment period. Doses were given within ten minutes of the scheduled time for each subject.

All feces eliminated after consumption of the first controlled meal were collected as individual samples in tared collection containers, labeled, accurately weighed, then frozen and stored at or below -20°C. Individual stools during each twenty four hour interval were collected and stored separately. Fecal collections from Days 5 and 11 were submitted for analysis of calcium, magnesium, and potassium.

Every urine specimen was collected and weighed. For Study Days 5 and 11 the urine is pooled for that day and analyzed for K, Mg, and Ca.

Change in fecal metal excretion (e.g., sodium, potassium, magnesium and calcium) for varying administrations of CLP are shown in Table 16.

**Table 16: Change in Fecal Metal Excretion (mg/day)**

| | **Na** | **K** | **Mg** | **Ca** |
|---|---|---|---|---|
| Milled, uncoated | 5.56 | 0.21 | 7.27 | 3.11 |
| Milled, uncoated; mannitol | 3.49 | -0.02 | 1.84 | 0.92 |
| Beads, uncoated; mannitol | 3.62 | 0.16 | 2.94 | 2.10 |
| Milled, coated pH 5.5 | 6.28 | 0.31 | 4.66 | 3.79 |
| Milled, coated pH 5.5; mannitol | 6.39 | 0.12 | 2.38 | 3.33 |

### Example 19

A clinical trial of an exemplary CLP was conducted. Objectives of the clinical trial included: to determine the safety, tolerability and degree of uptake by CLP of sodium, calcium, magnesium, potassium, iron, copper, zinc and phosphorous; and to determine amount of fluid absorbed per gram of CLP administered as assessed by stool weight compared between baseline and treatment periods.

Primary endpoints included: net sodium balance compared among treated and control groups. Secondary endpoints included: change in stool weight compared among treated and control groups; net balance of calcium, magnesium, potassium, iron, copper, zinc and phosphorous compared among treated and control groups; fluid consumed and excreted in the treated groups compared with the control group; and safety and tolerability based upon review of vital signs, clinical safety labs and adverse events.

For this clinical trial, an open-label, multiple-dose study was conducted in up to five end stage renal disease (ESRD) patients is conducted. All patients recieved CLP. Patients participated in a three day baseline period during which diet was controlled and stool weight, fecal electrolytes and fluid balance was determined. The treatment period began on Day 4. The patients selected had the ability to swallow up to 6 capsules for each of the study doses during each of the study days that include CLP dosing.

The dose of CLP was fifteen grams per day and given for times per day (QID). Based on the extent of sodium and other cation removal seen at fifteen grams per day in these ESRD patients, it may be decided to reduce or increase the dose of CLP. Due to the rate of effect, CLP was encapsulated in size 00 hydroxypropylmethylcellulose (HPMC) capsules. Five groups administered varying doses of CLP were tested (e.g., Group 1, 15 grams; Group 2, 25 grams; Group 3, 35 grams; Group 4, 45 grams; and Group 5, 55 grams).

During the 9 day treatment period, dosing with CLP capsules took place. Diet was controlled for the full twelve days of the study. A three day repeating meal schedule was administered throughout the study. Stool weight, fecal electrolytes and fluid balance were determined throughout the twelve day period.

CLP was administered one hour prior to each of four meals/snack and administered with water. The patients were requested to consume all of their meals. Meals that are not fully consumed were recorded as to the weight of each uneaten food item.

All urine was collected separately for each twenty four hour period, measured, and then pooled for the entire day to allow an aliquot to be sent for sodium, potassium, calcium, phosphorus and magnesium analysis. However, prior to pooling the afternoon specimen, the urine pH was determined on a two milliliter aliquot to be discarded.

All feces were collected in separate containers. Each sample was individually weighed and the color and consistency of the stool noted. The weights were added together to determine the total fecal weight resulting from each day's intake. The twenty four hour period for both stool and urine collection was considered from midnight to midnight.

CLP was supplied in hard, size 00 HPMC capsules and sent as a finished dosage from the Sponsor. To achieve the fifteen gram CLP dose, twenty-three capsules per day were divided as follows: six capsules administered one hour before each breakfast, lunch and dinner; five capsules administered one hour before each evening snack.

Oral doses of CLP were divided into four doses and administered for nine days, for a total of 36 consecutive doses. Doses were given within ten minutes of the scheduled time for each patient.

Patients were required to fast for at least eight hours at screening and four hours at admission prior to the collection of blood and urine samples for clinical laboratory tests. Fasting was not required prior to urine and blood samples taken during the study. Water ad libitum was allowed during the periods of fasting. Clinic staff monitored and recorded ingestion of the meals served during the study and any beverages, including water consumed, at the time the patients eat or drink.

All feces eliminated after consumption of the first controlled meal at dinner on Day 0 was collected as individual samples in tared collection containers, labeled, accurately weighed, then frozen and stored at or below -20°C. Individual stools during each twenty-four hour interval, starting at midnight, were collected and stored separately. Protocol number, patient number, study day, date and time of sample collection, and weight of sample and container were clearly and permanently indicated on collection containers for each individual stool. Sample time, color, consistency and weight was also recorded in the medical record. Fecal collections were submitted for analysis of sodium, calcium, magnesium, potassium, iron, copper, zinc and phosphorus.

Every urine specimen was collected and volume recorded. The urine was pooled for each day and analyzed for sodium, potassium, magnesium, calcium and phosphorous. A two milliliter aliquot from an afternoon specimen on Days 1 through 12 is sent for analysis of pH.

Changes in fecal metal excretion (*e.g*., sodium, potassium, magnesium and calcium) for varying administrations of CLP are shown in Table 17.

**Table 17: Change in Fecal Metal Excretion (mg/day)**

| **Dose (g)** | **Day of Trial** | **Na** | **K** | **P** | **Mg** | **Ca** |
|---|---|---|---|---|---|---|
| 7.5 | 1-3 | 204 | 1341 | 1866 | 380 | 2153 |
| 7.5 | 5-11 | 2265 | 4669 | 2145 | 619 | 3571 |
| 15 | 1-3 | 335 | 1957 | 2017 | 475 | 2413 |
| 15 | 5-11 | 4680 | 7556 | 2437 | 775 | 4497 |

### Example 20

Cross-linked polyelectrolyte polymer with bound counterions (CLP), including cross-linked polyacrylate polymeric beads with bound hydrogen counterions is used to treat kidney related diseases and disorders, such as end stage renal disease (ESRD). Such compositions may release and/or bind one or more ions (e.g., sodium,potassium) in the subject while other ion levels are kept constant. In addition, the CLP polymers may be useful to remove fluid from the subject.

Patients with end-stage renal failure (ESRD) due to a variety of causes (e.g., either on dialysis or with residual glomerular filtration) are treated. In an exemplary method, CLP is administered in an amount per day from about 1 gram to about 50 grams, for example, from about 4 grams to about 16 grams. The CLP may be administered 1, 2, 3, 4 or more times per day before or after eating.

CLP is administered in an amount effective to remove sodium and/or potassium. Additionally or alternatively, CLP is administered in an amount effect to reduce fluid in a patient. Further, the CLP may be administered in an effective amount to reduce blood pressure in a patient. Doses of CLP are administered before or after eating, for example, at the end of breakfast, lunch, and dinner. Increases in fecal or urinary sodium and/or potassium levels indicate the effectiveness of the administered doses. If there is a transient metabolic acidosis oral calcium carbonate, other phosphate binder, or alkali is administered to treat the acidosis. It is suggested that regardless of the agent selected, the initial extra alkali be calculated to provide 100 mEq (*e.g.,* 5g calcium carbonate, 8g of PhosLo, 44 g Revela, or 2g MgO).

The CLP may be administered alone or in combination with an agent for the treatment of ESRD, for example antihypertensives, vitamin D, acid blockers, antibiotics and/or narcotics. Additionally, the CLP may be co-administered with agents that enhance the ability of the CLP to remove absorb fluid from the gastrointestinal tract (e.g., small intestine), such as an osmotic agent. Additionally, or alternatively, the co-administered agent may increase the secretion of ions (such as sodium or potassium), into the intestine. Additionally, the CLP may also be co-administered with phosphate binders, such as aluminum hydroxide (*e.g*., Alucaps), calcium carbonate (*e.g*., Calcichew or Titralac), calcium acetate (*e.g*., Phosex or PhosLo), lanthanum carbonate (Fosrenol), or Sevelamer (*e.g*., Renagel or Renvela).

The CLP as described above is used to treat chronic kidney disease, congestive heart failure or hypertension.

While the present disclosure has been described and illustrated herein by references to various specific materials, procedures and examples, it is understood that the disclosure is not restricted to the particular combinations of material and procedures selected for that purpose. Numerous variations of such details can be implied as will be appreciated by those skilled in the art. It is intended that the specification and examples be considered as exemplary, only, with the true scope and spirit of the disclosure being indicated by the following claims. All references, patents, and patent applications referred to in this application are herein incorporated by reference in their entirety.

Further embodiments of the invention are enclosed herein:
1. A composition comprising cross-linked polyelectrolyte polymeric beads with bound counterions, with the proviso that when the bound counterions consist of sodium and hydrogen, the bound sodium counterions are less than 60% while the bound hydrogen ions are greater than 40% of the total bound counterions.
2. The method of item 1, wherein the beads absorb at least 20 fold of their mass in a saline solution.
2. The composition of item 1, wherein the counterion is an inorganic counterion.
3. The composition of item 2, wherein the inorganic counterion is selected from the group consisting of: hydrogen, sodium, potassium, calcium, magnesium and ammonium.
4. The composition of item 1, wherein the counterion is an organic counterion.
5. The composition of item 4, wherein the organic counterion is selected from the group consisting of: choline, arginine and lysine.
6. The composition of item 1, wherein at least one counterion is an inorganic counterion and at least one counterion is an organic counterion.
7. The composition of any one of items 3 or 6, wherein the inorganic counterion is hydrogen.
8. The composition of item 7, wherein hydrogen comprises from greater than 40% to 100% of the total bound counterions.
9. The composition of item 7, wherein hydrogen comprises about 100% of the total bound counterions.
10. The composition of item 7, wherein hydrogen comprises about 99% of the total bound counterions.
11. The composition of item 7, wherein hydrogen comprises about 95% of the total bound counterions.
12. The composition of item 7, wherein hydrogen comprises about 90% of the total bound counterions.
13. The composition of item 7, wherein hydrogen comprises about 50% of the total bound counterions.
14. The composition of any one of items 3 or 6, wherein the inorganic counterion is sodium.
15. The composition of item 3, wherein sodium comprises 5% or less of the total bound counterions.
16. The composition of item 3, wherein sodium comprises less than 25% of the total bound counterions.
17. The composition of any one of items 3 or 6, wherein the inorganic counterion is potassium.
18. The composition of item 17, wherein potassium comprises about 25%, 50% or 75% of the total bound counterions.
19. The composition of any one of items 3 or 6, wherein the inorganic counterion is ammonium.
20. The composition of item 19, wherein ammonium comprises about 25%, 50% or 75% of the total bound counterions.
21. The composition of any one of items 4 or 6, wherein the organic counterion is choline.
22. The composition of item 21, wherein choline comprises about 25%, 50% or 75% of the total bound counterions.
23. The composition of any one of items 4 or 6, wherein the organic counterion is lysine.
24. The composition of item 23, wherein lysine comprises about 25%, 50% or 75% of the total bound counterions.
25. The composition of item 1, wherein the counterions are hydrogen and sodium.
26. The composition of item 25, wherein hydrogen comprises from greater than 40% to 100% of total bound counterion and sodium comprises from greater than 0 to less than 60% of total bound counterion.
27. The composition of item 1, wherein the counterions are hydrogen and potassium.
28. The composition of item 1, wherein the counterions are sodium and potassium.
29. The composition of item 1, wherein the counterions are sodium, potassium and hydrogen.
30. The composition of item 1, wherein the counterions are hydrogen and ammonium.
31. The composition of item 1, wherein the counterions are hydrogen and choline.
32. The composition of item 1, wherein the counterions are hydrogen and lysine.
33. The composition of item 1, wherein the beads are substantially coated with a coating.
34. The composition of item 1, wherein the beads are surrounded by a capsule.
35. The composition of item 34, wherein the capsule is coated with a coating.
36. The composition of any one of items 33 or 35, wherein the coating is an enteric or delayed release coating.
37. The composition of item 1, wherein the polyelectrolyte is polyacrylate.
38. A method of modulating levels of more than one ions in a subject comprising administering an effective amount of the composition of any one of items 1-36 to the subj ect.
39. The method of item 38 further comprising identifying a subject in need of modulation of more than one ion.
40. The method of item 38, wherein the modulation is an increase or decrease in the level of one or more ions.
41. The method of item 38, wherein the composition binds to one or more ions in the subject thereby decreasing the levels of one or more ions in the subject.
42. The method of item 38, wherein the composition releases one or more ions in the subject thereby increasing the levels of one or more ions in the subject.
43. The method of item 38, wherein the composition binds to one or more first ions in the subject thereby decreasing the levels of one or more first ions in the subject and the composition releases one or more second ions in the subject thereby increasing the levels of one or more second ions in the subject.
44. The method of item 38, wherein the composition removes sodium.
45. The method of item 38, wherein the composition removes sodium and releases potassium.
46. The method of item 38, wherein the composition removes sodium and removes potassium.
47. The method of item 38, wherein the composition removes sodium and removes potassium without releasing hydrogen.
48. The method of item 38, wherein the cross-linked polyelectrolyte polymer is directly administered to the colon.
49. The method of item 38, wherein the cross-linked polyelectrolyte polymer is directly administered to the small intestine.
50. The method of item 49, wherein the polymer is directly administered to the jejunum.
51. A method of removing fluid from a subject comprising administering an effective amount of the composition of any one of items 1- 36 to the subject.
52. The method of item 51 further comprising identifying a subject in need of removal of the fluid.
53. The method of item 51 further comprising administering to the subject one or more agents that increase the amount of fluid in the intestine.
54. The method of item 53, wherein the agent is selected from the group consisting of: mannitol, polyethylene glycol and lubiprostone.
55. The method of item 54, wherein polyethylene glycol has a molecular weight between 400 and 10,000 Daltons.
56. The method of item 54, wherein the polyethylene glycol has a molecular weight between 400 and 4000 Daltons.
57. The method of item 51, wherein the composition is directly administered to the colon.
58. The method of item 51, wherein the composition is directly administered to the small intestine.
59. The method of item 58, wherein the composition is directly administered to the jejunum.
60. The method of item 51, wherein the composition is administered orally.
61. The method of item 51, wherein the subject has cardiac disease.
62. The method of item 61, wherein the cardiac disease is congestive heart failure.
63. The method of item 51, wherein the subject has kidney disease.
64. The method of item 63, wherein the kidney disease is nephrosis, nephritis, chronic kidney disease (CKD) or end stage renal disease (ESRD).
65. The method of item 51, wherein the subject has an intestinal or nutritional disorder.
66. The method of item 65, wherein the nutritional disorder is kwashiorkor or gluten-sensitive enteropathy.
67. The method of item 51, wherein the subject has hepatic disease.
68. The method of item 67, wherein the hepatic disease is ascites or cirrhosis of the liver.
69. The method of item 51, wherein the subject has an endocrine, neurological or immune system disorder.
70. The method of item 69, wherein the endocrine disorder is preclampsia or eclampsia.
71. The method of item 69, wherein the neurological disorder is angioneurotic edema.
72. A method of removing one or more waste products from a subject comprising: administering an effective amount of the composition of any one of items 1-36 to the subj ect.
73. The method of item 72 further identifying a subject in need of removal of one or more waste products;
74. The method of item 72, wherein the waste product is a metabolic waste.
75. The method of item 74, wherein the metabolic waste is urea, uric acid, creatinine, sodium or potassium.
76. The method of item 72 further comprising administering to the subject one or more agents that increase the amount of fluid in the intestine.
77. The method of item 76, wherein the agent is selected from the group consisting of: mannitol and lubiprostone.
78. The method of item 72, wherein the composition is directly administered to the colon.
79. The method of item 72, wherein the composition is directly administered to the small intestine.
80. The method of item 79, wherein the composition is directly administered to the jejunum.
81. The method of item 72, wherein the composition is administered orally.
82. A method of preparing cross-linked polyelectrolyte polymeric beads with 100% bound hydrogen counterion that absorb about 20-fold or more of their mass in a saline solution comprising:
   (a.) obtaining a cross-linked polyelectrolyte polymeric beads;
   (b.) treating the particles with an acid until 100% of bound counterions on the beads are hydrogen; and
   (c.) drying the washed beads.
83. The method of item 82, wherein the polyelectrolyte is polyacrylate.
84. The method of item 82 further comprising the step of disrupting the dried beads by milling.
85. The method of item 82 further comprising the step of mixing the disrupted beads with a solution comprising counterions other than hydrogen.
86. The method of item 82, wherein the counterions are selected from hydrogen, sodium, potassium, magnesium, calcium, ammonium, choline, arginine and lysine.
87. The method of item 82 further comprising the step of washing the disrupted beads.
88. The method of item 87 further comprising the step of drying the washed beads
89. The method of any one of items 82 or 88, wherein the beads are substantially coated.
90. The method of any one of items 82 or 88, wherein the beads are surrounded by a capsule.
91. The method of item 90, wherein the capsule is coated with a coating.
92. The method of any one of items 82 or 88, wherein the coating is an enteric or delayed release coating.
93. A method of preparing cross-linked polyelectrolyte beads comprising:
   (a.) polymerizing by cross-linked electrolyte monomers neutralized with sodium;
   (b.) obtaining cross-linked polyelectrolyte polymeric beads with bound sodium counterions from the polymerization of (a.); and
   (c.) reducing the percentage of bound sodium counterions on the beads.
94. A method of preparing cross-linked polyelectrolyte polymeric beads with less than 60% bound sodium counterion comprising:
   (a.) preparing cross-linked polyelectrolyte polymeric beads with greater than 60% bound sodium counterions;
   (b.) reducing the bound sodium counterions to less than 60%.
95. The method of item 93 further comprising (d.) disrupting the beads.
96. The method of item 95, wherein the disrupted beads have a particle size of 212-500 microns.
97. The method of item 95 further comprising (e.) encapsulating the disrupted beads in a capsule.
98. The method of item 93, wherein the monomers are acrylic acid.
99. The method of item 93, wherein the polyelectrolyte is polyacrylate.
100. The method of item 93, wherein monomers are neutralized from about 60% to about 100% with sodium.
101. The method of item 93, wherein the monomers are about 80% neutralized with sodium.
102. The method of item 93, wherein the polymerization is by a method of inverse suspension polymerization.
103. The method of any one of items 93 or 94, wherein the bound sodium counterions are reduced to less than 60%.
104. The method of any one of items 93 or 94, wherein the bound sodium counterions are reduced to less than 50%.
105. The method of any one of items 93 or 94, wherein the bound sodium counterions are reduced to less than 25%.
106. The method of any one of items 93 or 94, wherein the bound sodium counterions are reduced to less than 10%.
107. The method of any one of items 93 or 94, wherein the bound sodium counterions are reduced to less than 5%.
108. The method of any one of items 93 or 94, wherein the bound sodium counterions are reduced to less than 1%.
109. The method of any one of items 93 or 94, wherein the bound sodium counterions are reduced by acid treatment of the beads with acid.
110. The method of item 109, wherein the acid treated beads are washed to remove excess acid.
111. The method of item 110, wherein the washed beads are dried.
112. The method of item 111, wherein the beads are encapsulated in a capsule.
113. The method of item 112, wherein the capsule is substantially coated with a coating.
114. The method of item 111, wherein the beads are substantially coated with a coating.
115. The method of item 110, wherein the beads comprise disrupted beads.
116. A pharmaceutical composition comprising the cross-linked polyelectrolyte beads prepared by the methods of any one of items 93-115.
117. Cross-linked polyelectrolyte polymeric beads with bound counterions comprising 5% or less sodium bound counterions.
118. Cross-linked polyelectrolyte polymeric beads with bound counterions comprising 1% or less sodium bound counterions.
119. A method of treating a patient with end stage renal disease comprising administering to a patient with the disease cross-linked polyelectrolyte polymeric beads wherein the beads comprise less than 60%, less than 50%, less than 25%, less than 10%, less than 5% or less than 1% bound sodium.
120. A method of treating a patient with congestive heart failure comprising administering to a patient with the disease cross-linked polyelectrolyte polymeric beads wherein the beads comprise less than 60%, less than 50%, less than 25%, less than 10%, less than 5% or less than 1% bound sodium.
121. A method of treating a patient with chronic kidney disease comprising administering to a patient with the disease cross-linked polyelectrolyte polymeric beads wherein the beads comprise less than 60%, less than 50%, less than 25%, less than 10%, less than 5% or less than 1% bound sodium.
122. A method of treating a patient with hypertension comprising administering to the patient with the disease cross-linked polyelectrolyte polymeric beads wherein the beads comprise less than 60%, less than 50%, less than 25%, less than 10%, less than 5% or less than 1% bound sodium.
123. A method of modulating levels of more than one ions in a subject comprising administering a composition comprising cross-linked polyelectrolyte polymeric beads with bound counterions that absorb at least 20-fold of their mass in a saline solution, with the proviso that when the bound counterions consist of sodium and hydrogen, the bound sodium counterions are less than 60% while the bound hydrogen ions are greater than 40% of the total bound counterions to the subject.
124. A method of removing fluid from a subject comprising administering a composition comprising cross-linked polyelectrolyte polymeric beads with bound counterions that absorb at least 20-fold of their mass in a saline solution, with the proviso that when the bound counterions consist of sodium and hydrogen, the bound sodium counterions are less than 60% while the bound hydrogen ions are greater than 40% of the total bound counterions to the subject.
125. A method of removing one or more waste products from a subject comprising administering a composition comprising cross-linked polyelectrolyte polymeric beads with bound counterions that absorb at least 20-fold of their mass in a saline solution, with the proviso that when the bound counterions consist of sodium and hydrogen, the bound sodium counterions are less than 60% while the bound hydrogen ions are greater than 40% of the total bound counterions to the subject.
126. A composition comprising cross-linked polyelectrolyte polymeric beads with bound counterions wherein bound sodium counterions are less than 60% of the total bound counterions.
127. A composition comprising cross-linked polyelectrolyte polymeric beads with bound counterions wherein bound hydrogen counterions are greater than 40% of the total bound counterions.
128. A composition comprising cross-linked polyelectrolyte polymeric beads with bound counterions wherein bound sodium counterions are less than 60% of the total bound counterions and bound hydrogen counterions are greater than 40% of the total bound counterions.

## Claims

1. A composition comprising cross-linked polyelectrolyte polymeric beads with bound counterions, with the proviso that when the bound counterions consist of sodium and hydrogen, the bound sodium counterions are less than 60% while the bound hydrogen ions are greater than 40% of the total bound counterions, wherein the beads optionally absorb at least 20 fold of their mass in a saline solution.

2. The composition of claim 1, wherein the counterions are inorganic counterions and are optionally selected from the group consisting of hydrogen, sodium, potassium, calcium, magnesium and ammonium, or wherein the counterion are organic counterions and are optionally selected from the group consisting of choline, arginine and lysine, or wherein at least one counterion is an inorganic counterion and at least one counterion is an organic counterion.

3. The composition of claim 2, wherein the inorganic counterion is:
- hydrogen, wherein the hydrogen optionally comprises from greater than 40% to 100%, about 100%, about 99%, about 95%, about 90% or about 50% of the total bound counterions;
- sodium, wherein the sodium optionally comprises less than 25% or 5% or less of the total bound counterions;
- potassium, wherein the potassium optionally comprises about 25%, 50% or 75% of the total bound counterions; and/or
- ammonium, wherein the ammonium optionally comprises about 25%, 50% or 75% of the total bound counterions.

4. The composition of claim 2 or 3, wherein the organic counterion is choline and wherein the choline optionally comprises about 25%, 50% or 75% of the total bound counterions; and/or is lysine, wherein the lysine optionally comprises about 25%, 50% or 75% of the total bound counterions.

5. The composition of claim 1, wherein the beads are substantially coated with a coating or are surrounded by a capsule, wherein the capsule is optionally coated with a coating, and wherein the coating is optionally an enteric or delayed release coating.

6. The composition of claim 1, wherein the polyelectrolyte is polyacrylate.

7. The composition of any of claims 1-6 for use as a medicament.

8. The composition of any one of claims 1-6 for use in the treatment of a disease associated with ion imbalances in a subject.

9. The composition for use of claim 8, wherein:
- the composition binds to one or more ions in the subject thereby decreasing the levels of one or more ions in the subject, or
- the composition releases one or more ions in the subject thereby increasing the levels of one or more ions in the subject, or
- the composition binds to one or more first ions in the subject thereby decreasing the levels of one or more first ions in the subject and the composition releases one or more second ions in the subject thereby increasing the levels of one or more second ions in the subject,
wherein optionally the composition:
- removes sodium, or
- removes sodium and releases potassium, or
- removes sodium and removes potassium, or
- removes sodium and removes potassium without releasing hydrogen.

10. The composition of any one of claims 1-6 for use in a disease associated with increased retention of fluid, optionally further comprising administering to the subject one or more agents that increase the amount of fluid in the intestine, wherein the agent is optionally selected from the group consisting of: mannitol, polyethylene glycol and lubiprostone, wherein the polyethylene glycol optionally has a molecular weight between 400 and 10,000 Daltons or between 400 and 4000 Daltons.

11. The composition for use of any or claims 7-10, wherein the composition is directly administered to the colon or to the small intestine, or to the jejunum, or is administered orally.

12. The composition for use of claim 10, wherein the disease is:
- cardiac disease, wherein the cardiac disease is optionally congestive heart failure or hypertension, or
- kidney disease, wherein the kidney disease is optionally nephrosis, nephritis, chronic kidney disease (CKD) or end stage renal disease (ESRD), or
- an intestinal or nutritional disorder, wherein the nutritional disorder is optionally kwashiorkor or gluten-sensitive enteropathy, or
- hepatic disease, wherein the hepatic disease is optionally ascites or cirrhosis of the liver, or
- an endocrine, neurological or immune system disorder, wherein the endocrine disorder is optionally preclampsia or eclampsia, or wherein the neurological disorder is optionally angioneurotic edema.

13. A method of preparing cross- linked polyacrylate polymers with 100% bound hydrogen counterion that absorb about 20-fold or more of their mass in a saline solution comprising:
(a) obtaining a cross-linked polyelectrolyte polymer;
(b) treating the polymer with an acid until 100% of bound counterions on the polymer are hydrogen; and
(c) drying the washed polymer,
optionally further comprising the step of disrupting the dried polymer by milling and optionally further comprising the step of mixing the disrupted polymer with a solution comprising counterions other than hydrogen and/or optionally further comprising the step of washing the disrupted polymer and/or optionally further comprising the step of drying the washed polymer.

14. The method of claim 13, wherein the polymer is substantially coated and/or wherein the polymer is surrounded by a capsule, wherein the capsule is optionally coated with a coating, wherein the coating is optionally an enteric or delayed release coating.

15. A method of preparing cross-linked polyacrylate polymer comprising:
(a.) polymerizing by cross-linked electrolyte monomers neutralized with sodium, optionally by a method of inverse suspension polymerization;
(b.) obtaining cross-linked polyacrylate polymer with bound sodium counterions from the polymerization of (a.); and
(c.) reducing the percentage of bound sodium counterions on the polyacrylate polymer,
wherein the bound sodium counterions are reduced to less than 10%, optionally to less than 5 % or less than 1% and
wherein the bound sodium counterions are reduced by acid treatment of the polymer with acid, and
optionally further comprising (d.) disrupting the polymer, wherein the disrupted polymer optionally has a particle size of 212-500 microns, and
optionally further comprising (e.) encapsulating the disrupted polymer in a capsule.
